# EUROPEAN PATENT SPECIFICATION

(11) **EP 4 377 295 B1**
(45) Date of publication and mention of the grant of the patent: **11.06.2025**
(21) Application number: 22754863.3
(22) Date of filing: 25.07.2022
(51) Int. Cl.: C07D 207/16, A61P 25/28, A61K 31/40

(54) **HALOACETHYDRAZIDES USEFUL AS AEP INHIBITORS**
HALOACETHYDRAZIDE VERWENDBAR ALS AEP-INHIBITOREN
HALOACÉTHYDRAZIDES UTILES EN TANT QU'INHIBITEURS D'AEP

(30) Priority: 28.07.2021 EP 21188258
(43) Date of publication of application: 05.06.2024
(73) Proprietor: F. Hoffmann-La Roche AG, 4070 Basel (CH)
(72) Inventor: BARTELS, Bjoern, 4070 Basel (CH); BAUMANN, Karlheinz, 4070 Basel (CH); GRUENINGER, Fiona, 4070 Basel (CH); HOCHSTRASSER, Remo, 4070 Basel (CH); KUHN, Bernd, 4070 Basel (CH); MORANDI, Federica, 4070 Basel (CH); PINARD, Emmanuel, 4070 Basel (CH)
(74) Representative: Jochnowitz, Evan
(86) International application number: PCT/EP2022/070752
(87) International publication number: WO 2023/006645

(56) References cited:
- WO-A1-2019/186164
- WO-A2-01/90063
- LEE JIYOUN ET AL: "Synthesis and evaluation of aza-peptidyl inhibitors of the lysosomal asparaginyl endopeptidase, legumain", BIOORGANIC & MEDICINAL CHEMISTRY LETTERS, vol. 22, no. 3, 21 December 2011 (2011-12-21), pages 1340 - 1343, XP028886997, ISSN: 0960-894X, DOI: 10.1016/J.BMCL.2011.12.079

## Description

The present invention relates to organic compounds useful for therapy and/or prophylaxis in a patient, and in particular to compounds that inhibit AEP activity.

The present invention provides novel compounds of formula I 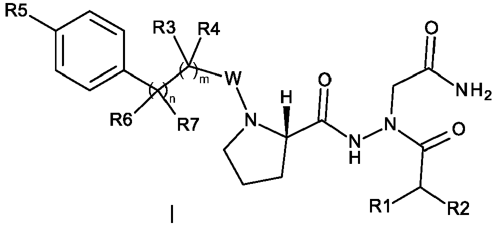 wherein
R¹ is halo or alkyl;
R² is H or halo;
R³ is H or halo;
R⁴ is H or halo;
or R³ and R⁴ form a 3-membered or a 4-membered cycloalkyl ring;
R⁵ is H, halo, haloalkoxy, alkynyl, alkynylalkoxy or alkoxyphenyl;
R⁶ is H
R⁷ is H
W is -C(O)- or -S(O)₂-;
m is 0 or 1;
n is 0 or 1;
and pharmaceutically acceptable salts.

Furthermore, the invention includes all racemic mixtures, all their corresponding enantiomers and/or optical isomers.

### Background of the Invention

### Alzheimer's disease (AD)

Alzheimer's disease is the most common form of dementia occurring primarily in the elderly and affecting over 5 million people in the US alone. This number is expected to triple by 2050. The prevalence of AD is age-related and patients frequently require institutionalization during the later stages of the disease. Because of its severity, increasing prevalence, long duration and high cost of care, AD will continue to represent a major public health issue in coming years.

Currently approved therapies for AD modulate neurotransmission (e.g. Aricept, an acetylcholinesterase inhibitor). These drugs give short-term relief from some symptoms but do not change the underlying pathology or the course of the disease. There is a significant unmet medical need for more effective treatments for AD at all stages and in particular, for novel treatments that slow or delay progression of the disease.

There are two distinct major histopathological lesions in AD brain, *viz*. amyloid plaques comprising aggregated Aβ peptide and neurofibrillary tangles comprising aggregated tau protein. Extracellular Aβ peptide accumulation is thought to be the initial event in a cascade of pathological changes that includes tau aggregation and neuronal loss and culminating in dementia (amyloid cascade hypothesis, Selkoe and Hardy, EMBO Mol Med 2016).

The most advanced drugs in development for AD aim to modify disease progression by lowering Aβ. These drugs include Aβ-specific antibodies, which promote Aβ clearance, as well as small-molecule inhibitors and modulators of the proteolytic enzymes responsible for Aβ generation i.e. β- and γ-secretase. Strategies to mitigate tau aggregation are needed to complement Aβ-lowering approaches. Several companies are pursuing tau-directed monoclonal antibodies for AD. However, tau antibodies are still in early-stage clinical development are unlikely to emerge as standard of care for AD in the next few years.

AD belongs to a larger group of neurodegenerative, dementing illnesses known as tauopathies. This group of diseases includes fronto-temporal dementia (FTD-MAPT), progressive supranuclear palsy (PSP) and corticobasal degeneration (CBD). The common feature of tauopathies is the presence of intracellular, fibrillary tau aggregates. Therapeutic approaches that target tau in AD may be applicable to primary tauopathies. There are no approved drugs for specific treatment of non-AD tauopathies.

### Tau aggregation and Alzheimer's disease

The presence of intraneuronal neurofibrillary tangles is one of the defining pathologies of AD. The main component of tangles, tau protein, is normally a highly soluble protein that associates with and stabilizes microtubules. In AD and other tauopathies, tau undergoes structural changes that cause it to aggregate (Vaquer-Alicea et al, Acta Neuropath 2021). Tau aggregation is central to disease development in tauopathies as demonstrated by multiple lines of evidence:
(1) Many frontotemporal dementia-linked *MAPT* mutations promote tau fibril assembly;
(2) Cognitive ability of AD patients correlates inversely with tau aggregate burden;
(3) Multimodal imaging of AD patients shows that tau aggregate deposition correlates with regional brain atrophy and dysfunction;
(4) Injection of fibrillar tau but not monomeric tau causes development of tangles in mouse brain;
(5) Tau added exogenously to cells must be aggregated in order to "seed" intracellular tau pathology.

Thus, tau aggregation is a toxic-gain-of-function in disease. Prevention of tau aggregation is therefore expected to protect against neurodegeneration in AD and primary tauopathies.

### Tau truncation by AEP

The microtubule-binding region (MBTR) of tau is the key part of the molecule that nucleates fibril assembly. Structural studies have shown that the centrally located MBTR is normally covered by the N- and C-terminal regions of the molecule, thereby precluding tau-tau interactions in solution. Post-translational modifications such as phosphorylation and proteolytic cleavage open up the tau molecule, expose MBTR and promote aggregation. PHF tau isolated from AD brain comprises a large proportion of truncated tau species, suggesting that tau truncation is integral to the tau aggregation process.

The lysosomal cysteine proteinase AEP/legumain has been shown to cleave tau on either side of the MBTR, thereby exposing the MBTR and promoting aggregation. Stress-induced upregulation of AEP activity in primary mouse neurons promotes tau truncation. Overexpression of AEP-derived tau fragment 1-368 in neurons is strongly neurotoxic, whereas overexpression of full-length tau is not. In addition to cleaving tau, AEP may indirectly influence tau aggregation by promoting tau phosphorylation: AEP is known to (indirectly) inhibit protein phosphatase 2A, the key enzyme regulating dephosphorylation of tau.

### AEP in AD

Published data show that AEP is upregulated in AD (Zhang et al, Nat Med 2014). Other authors have shown that AEP is overactivated in AD (Wang et al, Mol Cell 2017; Basurto-Islas et al, J Biol Chem 2013). Whether due to upregulation or overactivation of AEP, the AEP-cleaved tauN368 fragment is enriched in AD brain tissue (Zhang et al, Nat Med 2014). The TauN368/total-Tau ratio was significantly decreased in CSF from AD patients and strongly correlated negatively with 18F-GTP1 tau PET signal (Blennow et al, Brain 2020).

AEP may contribute to AD pathogenesis beyond promoting tau aggregation. The AEP-cleaved fragment tauN368 was recently shown to augment BACE1 expression and Aβ production via binding to the BACE1 transcription factor STAT1 (Zhang et al, Mol Psych Further reference can be made to: Lee Jiyoun et al., Bioorganic & Medicinal Chemistry Letters, 2283), 2011, pages 1340-1343; WO2019/186164; WO01/90063.

### Summary of the invention

The present invention provides novel compounds of formula I: 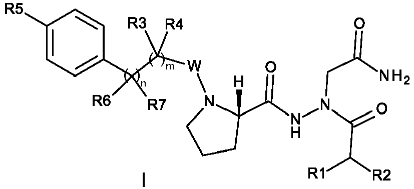 wherein
R¹ is halo or alkyl;
R² is H or halo;
R³ is H or halo;
R⁴ is H or halo;
or R³ and R⁴ form a 3-membered or a 4-membered cycloalkyl ring;
R⁵ is H, halo, haloalkoxy, alkynyl, alkynylalkoxy or alkoxyphenyl;
R⁶ is H
R⁷ is H
W is -C(O)- or -S(O)₂-;
m is 0 or 1;
n is 0 or 1;
and pharmaceutically acceptable salts.

The term "alkyl" denotes a monovalent linear or branched saturated hydrocarbon group of 1 to 6 carbon atoms. In some embodiments, if not otherwise described, alkyl comprises 1 to 6 carbon atoms (C₁₋₆-alkyl), or 1 to 4 carbon atoms (C₁₋₄-alkyl). Examples of C₁₋₆-alkyl include methyl, ethyl, propyl, isopropyl, n-butyl, iso-butyl, sec-butyl, tert-butyl and pentyl. Particular alkyl groups include methyl and ethyl. Preferred alkyl group is methyl. When an alkyl residue having a specific number of carbons is named, all geometric isomers having that number of carbons may be encompassed. Thus, for example, "butyl" can include n-butyl, sec-butyl, isobutyl and t-butyl, and "propyl" can include n-propyl and isopropyl.

The term "alkoxy" denotes a group of the formula -O-R', wherein R' is a C₁₋₆-alkyl group. Examples of C₁₋₆-alkoxy groups include methoxy, ethoxy, n-propoxy, isopropoxy, n-butoxy, isobutoxy and tert-butoxy. Particular examples are methoxy and ethoxy. Preferred example is methoxy.

The term "alkynyl" refers to an unsaturated unbranched or branched univalent hydrocarbon chain having at least one site of acetylenic unsaturation (that is, having at least one moiety of the formula C≡C). In some embodiments, unless otherwise specified, alkynyl comprises 2 to 6 carbon atoms, or 2 to 4 carbon atoms. Examples of alkynyl groups include, but are not limited to, ethynyl (or acetylenyl), prop-1-ynyl, prop-2-ynyl (or propargyl), but-1-ynyl, but-2-ynyl, and but-3-ynyl. Particular example is ethynyl.

The term "alkynylalkoxy" denotes a C₁₋₆ alkoxy group wherein at least one of the hydrogen atoms of the C₁₋₆ alkoxy groups is replaced by a C₂₋₆ alkynyl group. Particular examples are ethynylmethoxy, ethynylethoxy, prop-2-ynoxy, propynylmethoxy, propynylethoxy. Particular example is prop-2-ynoxy.

The term "alkoxyphenyl" denotes a phenyl substituted by an alkoxy group as defined above at ortho, meta or para position. Particular example is 4-methoxyphenyl.

The term "cycloalkyl" denotes monocyclic or polycyclic saturated or partially unsaturated, non-aromatic hydrocarbon. In some embodiments, unless otherwise described, cycloalkyl comprises 3 to 8 carbon atoms, 3 to 6 carbon atoms, or 3 to 5 carbon atoms. In some embodiments, cycloalkyl is a saturated monocyclic or polycyclic hydrocarbon. In other embodiments, cycloalkyl comprises one or more double bonds (e.g., cycloalkyl fused to an aryl or heteroaryl ring, or a non-aromatic monocyclic hydrocarbon comprising one or two double bonds). Polycyclic cycloalkyl groups may include spiro, fused, or bridged polycyclic moieties wherein each ring is a saturated or partially unsaturated, non-aromatic hydrocarbon. Examples of cycloalkyl groups include cyclopropyl, cyclobutyl, cyclopentyl, cyclohexyl, octahydropentalenyl, spiro[3.3]heptanyl, and the like. Bicyclic means a ring system consisting of two saturated carbocycles having two carbon atoms in common. Examples for monocyclic cycloalkyl are cyclopropyl, cyclobutanyl, cyclopentyl, cyclohexyl or cycloheptyl. Particular examples are cyclopropyl and cyclobutanyl. Preferred example is cyclopropyl.

The term "halogen", "halide" and "halo" are used interchangeably herein and denote fluoro, chloro, bromo or iodo. Particular halogens are fluoro and chloro.

The term "haloalkoxy" denotes a C₁₋₆-alkoxy group wherein at least one of the hydrogen atoms of the C₁₋₆-alkoxy group has been replaced by the same or different halogen atoms. Examples of haloalkoxy are difluoromethoxy, trifluoromethoxy, difluoroethoxy and trifluoroethoxy. Particular example is trifluoromethoxy.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid, particularly hydrochloric acid, and organic acids such as formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and N-acetylcysteine. In addition, these salts may be prepared from addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyamine resins. The compound of formula I can also be present in the form of zwitterions. Particularly preferred pharmaceutically acceptable salts of compounds of formula I are the salts formed with formic acid and the salts formed with hydrochloric acid yielding a hydrochloride, dihydrochloride or trihydrochloride salt.

The abbreviation uM means micromolar and is equivalent to the symbol µM.

The abbreviation uL means microliter and is equivalent to the symbol µL.

The abbreviation ug means microgram and is equivalent to the symbol µg.

The compounds of formula I can contain several asymmetric centers and can be present in the form of optically pure enantiomers, mixtures of enantiomers such as, for example, racemates, optically pure diastereoisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

Also an embodiment of the present invention provides compounds according to formula I as described herein and pharmaceutically acceptable salts thereof, in particular compounds according to formula I as described herein and pharmaceutically acceptable salts thereof, more particularly compounds according to formula I as described herein.

An embodiment of the present invention provides compounds according to formula I as described here, wherein
R¹ is halo or alkyl;
R² is H or halo;
R³ is H or halo;
R⁴ is H or halo;
or R³ and R⁴ form a 3-membered or a 4-membered cycloalkyl ring;
R⁵ is H, halo, haloalkoxy, alkynyl, alkynylalkoxy or alkoxyphenyl;
R⁶ is H;
R⁷ is H;
W is -C(O)- or -S(O)₂-;
m is 0 or 1;
n is 0 or 1;
and pharmaceutically acceptable salts.

An embodiment of the present invention provides compounds according to formula I as described herein, wherein R¹ is halo.

An embodiment of the present invention provides compounds according to formula I as described herein, wherein R⁵ is H, halo or haloalkoxy.

An embodiment of the present invention provides compounds according to formula I as described herein, wherein W is -C(O)-.

An embodiment of the present invention provides compounds according to formula I as described herein, wherein m is 1 and n is 0.

An embodiment of the present invention provides compounds according to formula I as described herein, wherein R³ and R⁴ form a 3-membered or 4-membered cycloalkyl ring.

An embodiment of the present invention provides compounds according to formula I as described herein, wherein
R¹ is halo;
R² is H or halo;
R³ is halo;
R⁴ is halo;
or R³ and R⁴ form a 3-membered or a 4-membered cycloalkyl ring;
R⁵ is H, halo or haloalkoxyl;
W is -C(O)-;
m is 1;
n is 0;
and pharmaceutically acceptable salts.

An embodiment of the present invention provides compounds according to formula I as described herein, wherein
R¹ is halo;
R² is H or halo;
R³ and R⁴ form a 3-membered or a 4-membered cycloalkyl ring;
R⁵ is halo or haloalkoxyl;
W is -C(O)-;
m is 1;
n is 0;
and pharmaceutically acceptable salts.

An embodiment of the present invention provides compounds according to formula I as described herein, wherein
R¹ is F or Cl;
R² is H or F;
R³ and R⁴ form a 3-membered cycloalkyl ring;
R⁵ is chloro or trifluoromethoxy;
W is -C(O)-;
m is 1;
n is 0;
and pharmaceutically acceptable salts.

Particular examples of compounds of formula I as described herein are selected from
2-[(2-fluoroacetyl)-[[(2S)-1-(2,2-difluoro-2-phenyl-acetyl)pyrrolidine-2-carbonyl]amino]amino]acetamide;
2-[(2-fluoroacetyl)-[[(2S)-1-(3-phenylpropanoyl)pyrrolidine-2-carbonyl]amino]amino]acetamide;
2-[(2-fluoroacetyl)-[[(2S)-1-(1-phenylcyclobutanecarbonyl)pyrrolidine-2-carbonyl]amino]amino]acetamide;
2-[(2-fluoroacetyl)-[[(2S)-1-[1-(4-chlorophenyl)cyclobutanecarbonyl]pyrrolidine-2-carbonyl]amino]amino]acetamide;
2-[(2-fluoroacetyl)-[[(2S)-1-(2-phenylacetyl)pyrrolidine-2-carbonyl]amino]amino]acetamide;
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-(benzenesulfonyl)pyrrolidine-2-carbonyl]amino]amino]acetamide;
2-[(2-fluoroacetyl)-[[(2S)-1-(1-phenylcyclopropanecarbonyl)pyrrolidine-2-carbonyl]amino]amino]acetamide;
2-[(2-fluoroacetyl)-[[(2S)-1-benzylsulfonylpyrrolidine-2-carbonyl]amino]amino]acetamide;
2-[(2-fluoroacetyl)-[[(2S)-1-(2-phenylethylsulfonyl)pyrrolidine-2-carbonyl]amino]amino]acetamide;
2-[(2-fluoroacetyl)-[[(2S)-1-[1-(4-ethynylphenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[(2-fluoroacetyl)-[[(2S)-1-[4-(4-methoxyphenyl)phenyl]sulfonylpyrrolidine-2-carbonyl]amino]amino]acetamide;
2-[(2-fluoroacetyl)-[[(2S)-1-[1-(4-prop-2-ynoxyphenyl)cyclopropanecarbonyl]pyrrolidine-2-carbonyl]amino]amino]acetamide;
2-[((2R)-2-chloro-2-fluoro-acetyl)-[[(2S)-1-[4-(4-methoxyphenyl)phenyl]sulfonylpyrrolidine-2-carbonyl]amino]amino]acetamide;
2-[((2S)-2-chloro-2-fluoro-acetyl)-[[(2S)-1-[4-(4-methoxyphenyl)phenyl]sulfonylpyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[(2-chloroacetyl)-[[(2S)-1-[4-(4-methoxyphenyl)phenyl]sulfonylpyrrolidine-2-carbonyl]amino]amino]acetamide;
2-[(2-chloroacetyl)-[[(2S)-1-[1-[4-(trifluoromethoxy)phenyl]cyclopropanecarbonyl]pyrrolidine-2-carbonyl]amino]amino]acetamide;
2-[(2,2-dichloroacetyl)-[[(2S)-1-[1-[4-(trifluoromethoxy)phenyl]cyclopropanecarbonyl]pyrrolidine-2-carbonyl]amino]amino]acetamide;
2-[(2R)-2-chloropropanoyl-[[(2S)-1-[1-[4-(trifluoromethoxy)phenyl]cyclopropanecarbonyl]pyrrolidine-2-carbonyl]amino]amino]acetamide;
2-[(2S)-2-chloropropanoyl-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof

Further particular examples of formula I as described herein are selected from
2-[(2-fluoroacetyl)-[[(2S)-1-(2,2-difluoro-2-phenyl-acetyl)pyrrolidine-2-carbonyl]amino]amino]acetamide;
2-[(2-fluoroacetyl)-[[(2S)-1-[1-(4-chlorophenyl)cyclobutanecarbonyl]pyrrolidine-2-carbonyl]amino]amino]acetamide;
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[(2-chloroacetyl)-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof.

Most particular examples of formula I as described herein are selected from
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof.

Processes for the manufacture of compounds of formula I as described herein are an object of the invention.

### General Synthetic Schemes

Compounds of formula I, wherein R¹, R², R³, R⁴, R⁵, R⁶, R⁷, W, n, and m are as described above, can be accessed as depicted in Scheme 1. A compound of formula II, wherein R¹, and R² are as described above, can be reacted with a compound of formula III, wherein R³, R⁴, R⁵, R⁶, R⁷, W, n, and m are as described above, and, depending on the nature of W, X is an appropriate functional group, selected from F, Cl, Br, OH, O-N-Succinimidyl (OSu). Specifically, for W = -C(O)-, X can be selected from F, Cl, Br, OH, O-N-Succinimidyl, preferably from Cl, and OH. If X = OH, the reaction is carried out e.g. under standard amide coupling conditions known in the art. If X = F, Cl, Br, or OSu, the reaction can be carried out in the presence of stoichiometric amounts of an appropriate chemically inert base, e.g. a tertiary amine, such as triethyl amine, or diisopropylethyl amine, in a suitable polar aprotic or unpolar solvent, e.g. dimethylformamide, or dichloromethane. If W = -S(O)₂-, X can be selected from F, Cl, preferably Cl. The reaction is carried out in the presence of stoichiometric amounts of an appropriate chemically inert base, e.g. a tertiary amine, such as triethyl amine, or diisopropylethyl amine, in a suitable polar aprotic or unpolar solvent, e.g. dimethylformamide, or dichloromethane.

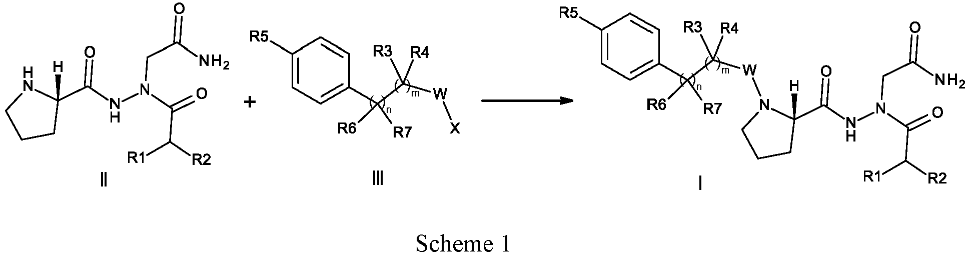

Alternatively, compounds of formula I, wherein R¹, R², R³, R⁴, R⁵, R⁶, R⁷, W, n, and m are as described above, can be accessed as depicted in Scheme 2. A compound of formula IV, wherein R³, R⁴, R⁵, R⁶, R⁷, W, n, and m are as described above, can be reacted with a compound of formula V, wherein R¹ and R² are as described above, and Y is an appropriate functional group, selected from F, Cl, Br, OH, O-N-Succinimidyl (OSu), preferably from Cl and OH. If Y = OH, the reaction is carried out e.g. under standard amide coupling conditions known in the art. If Y = F, Cl, Br, or OSu, the reaction can be carried out in the presence of stoichiometric amounts of an appropriate base, e.g. pyridine, in a suitable polar aprotic or unpolar solvent, e.g. dichloromethane.

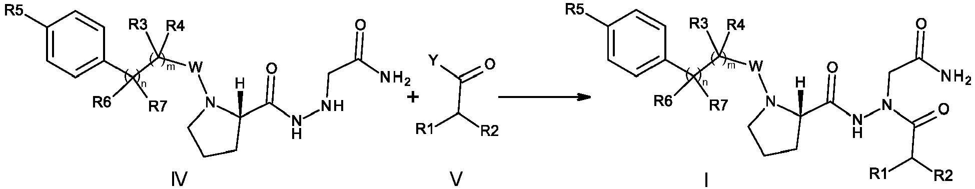

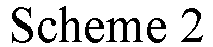

The intermediate compounds of formulae III and IV, wherein R³, R⁴, R⁵, R⁶, R⁷, W, n, and m are as described above, respectively, can be accessed by methods known in the art.

An embodiment of the present invention is a process to prepare a compound of formula I as defined above, comprising the reaction of a compound of formula II with a compound of formula III.

An embodiment of the present invention is a process to prepare a compound of formula I as defined above, comprising the reaction of a compound of formula IV with a compound of formula V.

Another embodiment of the invention provides a pharmaceutical composition or medicament containing a compound of the invention and a therapeutically inert carrier, diluent or excipient, as well as a method of using the compounds of the invention to prepare such composition and medicament. In one example, the compound of formula I may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula I is formulated in an acetate buffer, at pH 5. In another embodiment, the compound of formula I is sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

The compounds of formula I and their pharmaceutically acceptable salts can be processed with pharmaceutically inert, inorganic or organic adjuvants for the production of tablets, coated tablets, dragées,hard gelatin capsules, injection solutions or topical formulations Lactose, corn starch or derivatives thereof, talc, stearic acid or its salts etc. can be used, for example, as such adjuvants for tablets, dragées and hard gelatin capsules.

Suitable adjuvants for soft gelatin capsules, are, for example, vegetable oils, waxes, fats, semi-solid substances and liquid polyols, etc.

Suitable adjuvants for the production of solutions and syrups are, for example, water, polyols, saccharose, invert sugar, glucose, etc.

Suitable adjuvants for injection solutions are, for example, water, alcohols, polyols, glycerol, vegetable oils, etc.

Suitable adjuvants for suppositories are, for example, natural or hardened oils, waxes, fats, semi-solid or liquid polyols, etc.

Suitable adjuvants for topical ocular formulations are, for example, cyclodextrins, mannitol or many other carriers and excipients known in the art.

Moreover, the pharmaceutical preparations can contain preservatives, solubilizers, viscosity-increasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should it be appropriate. In the case of topical administration, the formulation can contain 0.001% to 15% by weight of medicament and the required dose, which can be between 0.1 and 25 mg in can be administered either by single dose per day or per week, or by multiple doses (2 to 4) per day, or by multiple doses per week It will, however, be clear that the upper or lower limit given herein can be exceeded when this is shown to be indicated.

The invention also relates in particular to:
A compound of formula I for use as therapeutically active substance;

Described but not claimed is a compound of formula I for use in the treatment of a disease modulated by AEP;

An embodiment of the present invention is a compound of formula I for use in the treatment or prophylaxis of Alzheimer's Disease, Primary age-related tauopathy (PART) dementia, Fronto-temporal dementia (FTD-MAPT), Chronic traumatic encephalopathy (CTE), Progressive supranuclear palsy (PSP), Corticobasal degeneration (CBD), Frontotemporal dementia and parkinsonism linked to chromosome 17 (FTDP-17), Lytico-bodig disease (Parkinson-dementia complex of Guam), Ganglioglioma and gangliocytoma, Meningioangiomatosis, Postencephalitic parkinsonism, Subacute sclerosing panencephalitis (SSPE), Pick's disease, or corticobasal degeneration.

An embodiment of the present invention is a compound of formula I for use in the treatment of prophylaxis of Alzheimer's disease, Primary age-related tauopathy (PART) dementia, Fronto-temporal dementia (FTD-MAPT), Chronic traumatic encephalopathy (CTE), Progressive supranuclear palsy (PSP), or Pick's disease.

An embodiment of the present invention is a compound of formula I for use in the treatment or prophylaxis of Alzheimer's disease.

Disclosed is the use of a compound of formula I for the preparation of a medicament for the treatment or prophylaxis of Alzheimer's Disease, Primary age-related tauopathy (PART) dementia, Fronto-temporal dementia (FTD-MAPT), Chronic traumatic encephalopathy (CTE), Progressive supranuclear palsy (PSP), Corticobasal degeneration (CBD), Frontotemporal dementia and parkinsonism linked to chromosome 17 (FTDP-17), Lytico-bodig disease (Parkinson-dementia complex of Guam), Ganglioglioma and gangliocytoma, Meningioangiomatosis, Postencephalitic parkinsonism, Subacute sclerosing panencephalitis (SSPE), Pick's disease, or corticobasal degeneration.

Disclosed is the use of a compound of formula I for the preparation of a medicament for the treatment or prophylaxis of Alzheimer's disease, Primary age-related tauopathy (PART) dementia, Fronto-temporal dementia (FTD-MAPT), Chronic traumatic encephalopathy (CTE), Progressive supranuclear palsy (PSP), or Pick's disease.

Disclosed is the use of a compound of formula I for the preparation of a medicament for the treatment or prophylaxis of Alzheimer's disease.

An embodiment of the present invention is a compound of formula I for use in the treatment or prophylaxis of Alzheimer's Disease, Primary age-related tauopathy (PART) dementia, Fronto-temporal dementia (FTD-MAPT), Chronic traumatic encephalopathy (CTE), Progressive supranuclear palsy (PSP), Corticobasal degeneration (CBD), Frontotemporal dementia and parkinsonism linked to chromosome 17 (FTDP-17), Lytico-bodig disease (Parkinson-dementia complex of Guam), Ganglioglioma and gangliocytoma, Meningioangiomatosis, Postencephalitic parkinsonism, Subacute sclerosing panencephalitis (SSPE), Pick's disease, or corticobasal degeneration.

An embodiment of the present invention is a compound of formula I for use in the treatment or prophylaxis of Alzheimer's disease, Primary age-related tauopathy (PART) dementia, Fronto-temporal dementia (FTD-MAPT), Chronic traumatic encephalopathy (CTE), Progressive supranuclear palsy (PSP), or Pick's disease.

An embodiment of the present invention is a compound of formula I for use in the treatment or prophylaxis of Alzheimer's disease.

Likewise an object of the present invention is a pharmaceutical composition comprising a compound according to formula I as described herein and a therapeutically inert carrier.

The invention will now be illustrated by the following examples, which have no limiting character.

In case the preparative examples are obtained as a mixture of enantiomers, epimers and/or diastereoisomers, the pure enantiomers can be obtained by methods known to those skilled in the art, such as e.g. chiral chromatography or crystallization.

### Abbreviations

The following abbreviations were used in the experimental part:
THF = tetrahydrofuran;
MTBE = methyl-tert-butylether;
DMF = dimethylformamide;
TLC = thin layer chromatography;
rt = room temperature, 20-25°C:
   Cbz = benzyloxycarbonyl;
   BOC = t-butyloxycarbonyl:
      HPLC = High Performance Liquid Chromatography;
      HBTU = Hexafluorophosphate Benzotriazole Tetramethyl Uronium;
      HATU = Hexafluorophosphate Azabenzotriazole Tetramethyl Uronium;

### Starting materials

Basic chemicals and solvents were purchased and used as is without further purification. Intermediates Int-1, Int-13, Int-14 are commercially available, or they can be synthesized using methods known in the art.

### INTERMEDIATES

### Intermediate 7: 2-[(2-Fluoroacetyl)-[[(2S)-pyrrolidine-2-carbonyl]amino]amino]acetamide

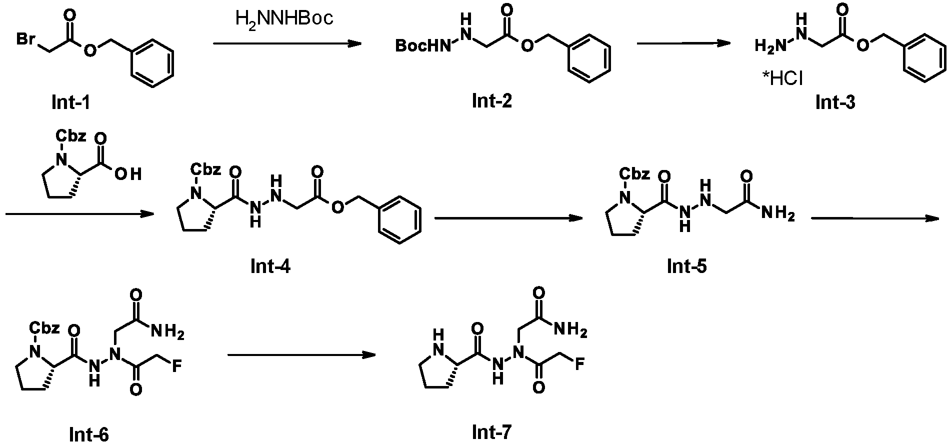

### Step 1: Benzyl 2-(2-tert-butoxycarbonylhydrazino)acetate (Int-2)

Tert-butyl carbazate (49761 mg, 376 mmol) and N,N-diisopropylethylamine (65 mL, 376 mmol) were dissolved in toluene (500 mL) and benzyl 2-bromoacetate (Int-1, 75.00 g, 327 mmol) was added. The mixture was stirred at 75 °C for 16 h. After cooling, the mixture was filtered and the filtrate was concentrated to give a yellow oil. The crude product was purified by column chromatography (silica gel, 220 g, petroleum ether / ethyl acetate, gradient 100:1 to 40:1 (v/v)) and the product containing fraction combined and concentrated in vacuo to give the title compound as colorless oil (60.00 g, 214 mmol, 65 % yield). MS m/z (ESI): 225.2 [M-CH2=C(CH3)2]⁺.

### Step 2: Benzyl 2-hydrazinoacetate hydrochloride (Int-3)

Benzyl 2-(2-tert-butoxycarbonylhydrazino)acetate (Int-2, 58.0 g, 207 mmol) was dissolved ethyl acetate (200 mL), and a solution of hydrogen chloride in ethyl acetate (4 M, 300 mL, 1200 mmol) was added. A large quantity of white precipitate was formed. The mixture was stirred at 25°C for 5 h. After that, the mixture was filtered and the solid was washed with ethyl acetate (200 mL) and dried in vacuo to give the title compound as white solid (42.0 g, 194 mmol, 94 % yield). MS m/z (ESI): 181.2 [M+H]⁺.

### Step 3: Benzyl (2S)-2-[[(2-benzyloxy-2-oxo-ethyl)amino]carbamoyl]pyrrolidine-1-carboxylate (Int-4)

Benzyl 2-hydrazinoacetate hydrochloride (Int-3, 1.10 g, 5.08 mmol) was dissolved in dichloromethane (15 mL), and Z-PRO-OH (1.27 g, 5.08 mmol), N,N-diisopropylethylamine (2.65 mL, 15.2 mmol) and N-[3-(dimethylamino)prop-1-yl]-N-ethylcarbodiimide hydrochloride (1.261 g, 6.60 mmol) were added at 0°C. The mixture was stirred at 25°C for 12 h. After that, the reaction was diluted with cold water (30 mL) and extracted with dichloromethane (2 x 15 mL). The combined organic layers were washed with brine (20 mL), dried over sodium sulfate and concentrated in vacuo to give a yellow oil. The crude product was purified by column chromatography (silica gel, 25 g, petroleum ether / ethyl acetate, gradient 10:1 to 1:2 (v/v)) to give the title compound as yellow oil (1800 mg, 4.37 mmol, 86 % yield). MS m/z (ESI): 412.3 [M+H]⁺.

### Step 4: Benzyl (2S)-2-[[(2-amino-2-oxo-ethyl)amino]carbamoyl]pyrrolidine-1-carboxylate (Int-5)

A solution of benzyl (2S)-2-[[(2-benzyloxy-2-oxo-ethyl)amino]carbamoyl]pyrrolidine-1-carboxylate (Int-4, 10.0 g, 24.3 mmol) in a solution of ammonia in methanol (7 N, 10 mL, 700 mmol) was stirred at 25 °C for 14 h. After that, the mixture was concentrated to give a light yellow oil. The light yellow oil was dissolved in water (100 mL) and extrated with methyltertbutyl ether (5 x 70 mL, every extraction process needed to still for 20 min because of the emulsification, a small amount of EtOH addition was beneficial to separation). The aqueous solution was lyophilized to give the title compound (15.9 mg, 49.6 mmol, 68% yield) as light yellow solid. MS m/z (ESI): 321.3 [M+H]⁺.

### Step 5: Benzyl (2S)-2-[[(2-amino-2-oxo-ethyl)-(2-fluoroacetyl)amino]carbamoyl]pyrrolidine-1-carboxylate (Int-6)

Benzyl (2S)-2-[[(2-amino-2-oxo-ethyl)amino]carbamoyl]pyrrolidine-1-carboxylate (Int-5, 4.20 g, 13.1 mmol) was dissolved in dichloromethane (35 mL) and the solution was cooled to -78°C (acetone / dry ice). Then, a solution of fluoroacetyl chloride (1645 mg, 17.0 mmol) in dichloromethane (15 mL) was added dropwise at -78°C. The mixture was stirred at -78°C for 0.5 h. The mixture was allowed to warm to 25°C and stirred for 1.5 h. After that, it was concentrated in vacuo to obtain the crude title compound as light yellow solid (4.90 g, 12.9 mmol, 98% yield), that was used in the next step without further purification. MS m/z (ESI): 403.2 [M+H]⁺.

### Step 6: 2-[(2-Fluoroacetyl)-[[(2S)-pyrrolidine-2-carbonyl]amino]amino]acetamide (Int-7)

Benzyl (2S)-2-[[(2-amino-2-oxo-ethyl)-(2-fluoroacetyl)amino]carbamoyl]pyrrolidine-1-carboxylate (Int-6, 3.90 g, 10.2 mmol) was dissolved in methanol (50 mL) and Palladium on charcoal (10%, 800 mg) was added. The resulting mixture was stirred under a hydrogen atmosphere (hydrogen balloon) at 25°C for 4 h. After that, the mixture was filtered through a pad of celite, washed with methanol (50 mL) and the filtrate was concentrated in vacuo to give the title compound as a light yellow solid (2.50 g, 10.1 mmol, 99% yield). MS m/z (ESI): 269.1 [M+H]⁺.

### Intermediate 12: 2-[2-[(2S)-1-[1-[4-(Trifluoromethoxy)phenyl]cyclopropanecarbonyl]pyrrolidine-2-carbonyl]hydrazino]acetamide

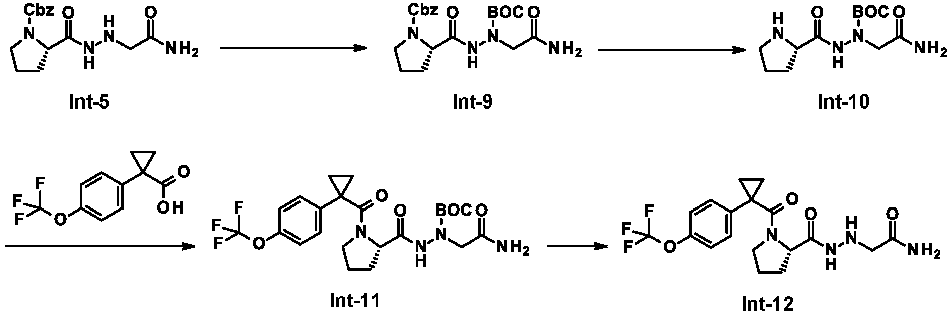

### Step 1: Benzyl (2S)-2-[[(2-amino-2-oxo-ethyl)-tert-butoxycarbonylamino]carbamoyl]pyrrolidine-1-carboxylate (Int-9)

Benzyl (2S)-2-[[(2-amino-2-oxo-ethyl)amino]carbamoyl]pyrrolidine-1-carboxylate (Int-5, 5.00 g, 15.6 mmol) was dissolved in dichloromethane (100 mL), and di-t-butyldicarbonate (4088 mg, 18.7 mmol) followed by triethylamine (4.73 g, 46.8 mmol) were added. The mixture was stirred at 25°C for 16 h. After that, the reaction mixture was concentrated in vacuo and the crude product purified via reversed phase chromatography. After lyophilization, the title compound was isolated as a white solid (3.00 g, 7.14 mmol, 46% yield). MS m/z (ESI): 421.1 [M+H]⁺.

### Step 2: tert-Butyl N-(2-amino-2-oxo-ethyl)-N-[[rac-(2S)-pyrrolidine-2-carbonyl]amino]carbamate (Int-10)

A mixture of benzyl (2S)-2-[[(2-amino-2-oxo-ethyl)-tert-butoxycarbonylamino]carbamoyl]pyrrolidine-1-carboxylate (Int-9, 1.50 g, 3.57 mmol) and Palladium on charcoal (10% m/m, 100 mg) in tetrahydrofuran (60 mL) was stirred at 25°C for 48 h under a hydrogen atmosphere (ballon). After that, the mixture was filtered over a pad of celite, washed with tetrahydrofuran (50 mL), and the filtrate concentrated in vacuo to afford the title compound as a white solid (1.00 g, 3.49 mmol, 98% yield).

### Step 3: tert-Butyl N-(2-amino-2-oxo-ethyl)-N-[[(2S)-1-[1-[4-(trifluoromethoxy)phenyl]cyclopropanecarbonyl]pyrrolidine-2-carbonyl]amino]carbamate (Int-11)

Tert-butyl N-(2-amino-2-oxo-ethyl)-N-[[ (2S)-pyrrolidine-2-carbonyl]amino]carbamate (Int-10, 120 mg, 0.420 mmol) was dissolved in dimethylformamide (2 mL), and HATU (148 mg, 0.630 mmol), diisopropylethylamine (163 mg, 1.26 mmol), and 1-[4-(trifluoromethoxy)phenyl]cyclopropane carboxylic acid (103 mg, 0.420 mmol) were added. The mixture was stirred at 25°C for 1 h. After that, the reaction mixture was purified via reversed phase chromatography (Phenomenex luna C18 150*25mm* 10um, eluent (water + 0.225% formic acid) / acetonitrile). After lyophilization, the title compound was obtained as white solid (100 mg, 0.190 mmol, 46% yield). MS m/z (ESI): 515.1 [M+H]⁺.

### Step 4: 2-[2-[(2S)-1-[1-[4-(Trifluoromethoxy)phenyl]cyclopropanecarbonyl]pyrrolidine-2-carbonyl]hydrazino]acetamide (Int-12)

tert-Butyl N-(2-amino-2-oxo-ethyl)-N-[[(2S)-1-[1-[4-(trifluoromethoxy)phenyl]cyclopropanecarbonyl]pyrrolidine-2-carbonyl]amino]carbamate (Int-11, 50 mg, 0.10 mmol) was dissolved in dichloromethane (0.3 mL) and trifluoroacetic acid (0.1 mL) was added. The mixture was stirred at 25°C for 1h. After that, the reaction mixture was concentrated in vacuo to afford the crude title compound as white solid (40 mg, 0.10 mmol, 99% yield), that was used in the next step without further purification. MS m/z (ESI): 415.1 [M+H]⁺.

### Intermediate 18: 2-[(2-Fluoroacetyl)-[[(2S)-pyrrolidine-2-carbonyl]amino]amino]acetamide

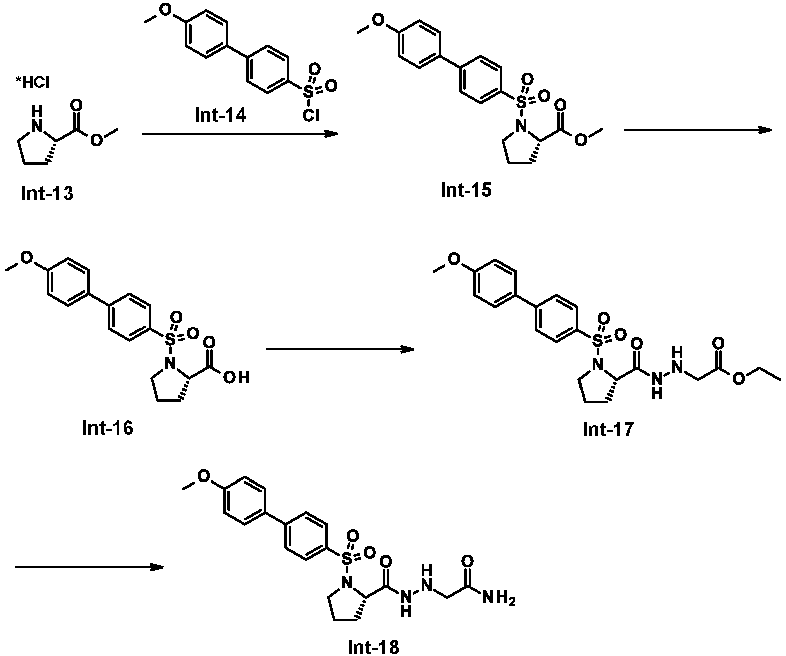

### Step 1: Methyl (2S)-1-[4-(4-methoxyphenyl)phenyl]sulfonylpyrrolidine-2-carboxylate (Int-15)

Methyl L-prolinate hydrochloride (1.34 g, 7.93 mmol, Int-13) was dissolved in dichloromethane (25 mL) and the solution was cooled to 0 °C (ice bath). Then, pyridine (3.1 g, 3.2 mL, 39.6 mmol) was added, followed by dropwise addition of a solution of 4'-methoxy-[1,1'-biphenyl]-4-sulfonyl chloride (2.69 g, 9.51 mmol, Int-14) in dichloromethane (25 mL). The yellow reaction mixture was stirred at 0° - 23 °C for 3 h. After that, the reaction mixture was poured into icecold aqueous citric acid solution (5% m/m, 50 mL), after phase separation the aqueous layer was extracted with dichloromethane (3 x 50 mL), the combined organics were dried over sodium sulfate, filtered, and the filtrate was concentrated in vacuo. The residue was purified by column chromatography (silica gel, 80 g, n-heptane / ethyl acetate, gradient 100:0 to 20:80 (v/v)) to yield, after concentration of product containing fractions, the title compound as a white solid (2.55 g, 6.79 mmol, 86 % yield). MS m/z (ESI): 376.1 [M+H]⁺.

### Step 2: (2S)-1-[4-(4-Methoxyphenyl)phenyl]sulfonylpyrrolidine-2-carboxylic acid (Int-16)

Methyl ((4'-methoxy-[1,1'-biphenyl]-4-yl)sulfonyl)-L-prolinate (2.52 g, 6.71 mmol, Int-15) was dissolved in a mixture of tetrahydrofurane (20 mL), methanol (5 mL) and water (10 mL). Lithium hydroxide monohydrate (321 mg, 7.62 mmol) was added and the reaction mixture was stirred at room temperature (23 °C) for 2 h. The mixture was acidified slowly by addition of aqueous citric acid (5% m/m, until pH 3 - 4), extracted with ethyl acetate (2 x 20 mL), the combined organic layers were dried over sodium sulfate, and concentrated in vacuo to provide the title compound as a white solid (2.40 g, 6.64 mmol, 99 % yield). No further purification. MS m/z (ESI): 362.1 [M+H]⁺.

### Step 3: 2-[2-[(2S)-1-[4-(4-Methoxyphenyl)phenyl]sulfonylpyrrolidine-2-carbonyl]hydrazino]acetamide (Int-17)

A suspension of ((4'-methoxy-[1,1'-biphenyl]-4-yl)sulfonyl)-L-proline (100 mg, 277 µmol, Int-16) and 1-hydroxybenzotriazole hydrate (56.8 mg, 360 µmol) in dichloromethane (2 mL) was cooled to 0 °C (ice bath). 1-(3-Dimethylaminopropyl)-3-ethylcarbodiimide (70.4 mg, 360 µmol) was added and the stirring was continued at 0 °C for 1 h. Then, ethyl aminoglycinate hydrochloride (85.6 mg, 553 µmol) and N,N-diisopropylethylamine (95 mg, 125 µl, 719 µmol) were added. The reaction mixture was allowed to warm to room temperature (23 °C) and stirred for 30 min. After that, it was poured into water (5 mL) and extracted with ethyl acetate (2 x 10 mL). The combined organic layers were washed with saturated aqueous sodium hydrogencarbonate solution (20 mL), dried over sodium sulfate, and concentrated in vacuo. The crude material was purified by column chromatography (silica gel, 12 g, n-heptane / ethyl acetate, gradient 100:0 to 0:100 (v/v)) to obtain the title compound as colourless gum (115 mg, 249 µmol, 90 % yield). MS (ESI) m/z: 462.2 [M+H]⁺.

### Step 4: (S)-2-(2-(((4'-Methoxy-[1,1'-biphenyl]-4-yl)sulfonyl)prolyl)hydrazineyl)acetamide (Int-18)

A solution of ethyl (S)-(1-((4'-methoxy-[1,1'-biphenyl]-4-yl)sulfonyl)pyrrolidine-2-carboxamido)glycinate (505 mg, 1.09 mmol, Int-17) in dimethylformamide (500 µL) was cooled to 0°C (ice bath). Ammonia (7 M in methanol, 15.6 mL, 109 mmol) was added, followed by sodium cyanide (5.4 mg, 109 µmol). The reaction mixture was stirred for 2 h at 0 °C, and for 15 h at room temperature (23 °C). After that, the reaction mixture was concentrated in vacuo, poured into water / brine (1:1 v/v, 10 mL) and extracted with dichloromethane / methanol (9:1 v/v, 2 x 20 mL). The combined organic layers were dried over sodium sulfate and concentrated in vacuo to yield the title compound as a white solid (454 mg, 1.05 mmol, 96 % yield). MS (ESI) m/z: 433.2 [M+H]⁺.

### EXAMPLES

### Example 1

### 2-[(2-Fluoroacetyl)-[[(2S)-1-(2,2-difluoro-2-phenyl-acetyl)pyrrolidine-2-carbonyl]amino]amino]acetamide

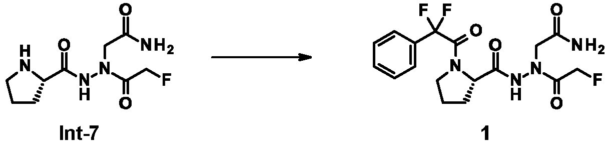

2-[(2-Fluoroacetyl)-[[(2S)-pyrrolidine-2-carbonyl]amino]amino]acetamide (Int-7, 206 mg, 0.840 mmol) was dissolved in dimethylformamide (1.5 mL), and 2,2-difluoro-2-phenylacetic acid (80 mg, 0.46 mmol), HBTU (211 mg, 0.56 mmol), and N-methylmorpholine (94 mg, 0.93 mmol) were added. The mixture was stirred at 25°C for 4 h. After that, it was purified directly by preparative HPLC (Shim-pack C18 150*25*10um, eluent (water + 0.225% formic acid) / acetonitrile, gradient 76:24 to 56:44 within 10 min). After lyophilization, the title compound was obtained as white solid (30 mg, 0.07 mmol, 16% yield). MS (ESI) m/z: 401.2 [M+H]⁺.

### Example 2

### 2-[(2-Fluoroacetyl)-[[(2S)-1-(3-phenylpropanoyl)pyrrolidine-2-carbonyl]amino]amino]acetamide

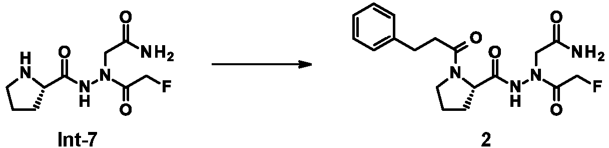

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), and 3-phenylpropionic acid (30.5 mg, 0.20 mmol), HBTU (92 mg, 0.24 mmol), and N-methylmorpholine (41 mg, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile, gradient 81:19 to 51:49 within 11 min). After lyophilization, the title compound was isolated as white solid (13 mg, 0.03 mmol, 17% yield). MS (ESI) m/z: 379.1 [M+H]⁺.

### Example 3

### 2-[(2-Fluoroacetyl)-[[(2S)-1-(1-phenylcyclobutanecarbonyl)pyrrolidine-2-carbonyl]amino]amino]acetamide

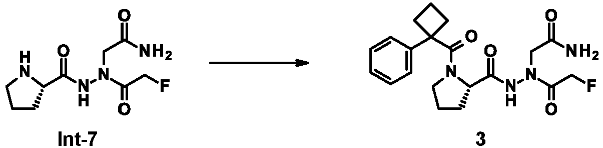

2-[(2-Fluoroacetyl)-[[(2S)-pyrrolidine-2-carbonyl]amino]amino]acetamide (Int-7, 84 mg, 0.34 mmol) was dissolved in dimethylformamide (1.5 mL), 1-phenylcyclobutanecarboxylic acid (50 mg, 0.28 mmol), HATU (129 mg, 0.34 mmol), and N,N-diisopropylethylamine (0.1 mL, 0.57 mmol) were added. The mixture was stirred at 25°C for 12 h. After that, it was purified directly by preparative HPLC (Phenomenex luna C18 150*25mm* 10um, eluent (water + 0.225% formic acid) / acetonitrile). After lyophilization, the title compound was obtained as white solid (22 mg, 0.05 mmol, 18% yield). MS (ESI) m/z: 405.2 [M+H]⁺.

### Example 4

### 2-[(2-Fluoroacetyl)-[[(2S)-1-[1-(4-chlorophenyl)cyclobutanecarbonyl]pyrrolidine-2-carbonyl]amino]amino]acetamide

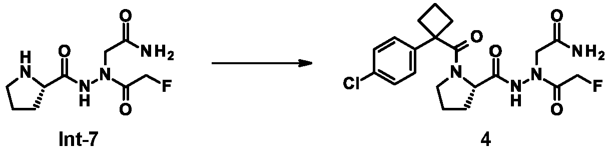

2-[(2-Fluoroacetyl)-[[(2S)-pyrrolidine-2-carbonyl]amino]amino]acetamide (Int-7, 70 mg, 0.28 mmol) was dissolved in dimethylformamide (1.5 mL), 1-(4-chlorophenyl)-1-cyclobutanecarboxylic acid (50 mg, 0.24 mmol), HATU (108 mg, 0.28 mmol), and N,N-diisopropylethylamine (0.04 mL, 0.24 mmol) were added. The mixture was stirred at 25°C for 12 h. After that, it was purified directly by preparative HPLC (Phenomenex luna C18 150*25mm* 10um, eluent (water + 0.225% formic acid) / acetonitrile). After lyophilization, the title compound was obtained as white solid (23 mg, 0.05 mmol, 21% yield). MS (ESI) m/z: 461.2 [M+Na]⁺.

### Example 5

### 2-[(2-Fluoroacetyl)-[[(2S)-1-(2-phenylacetyl)pyrrolidine-2-carbonyl]amino]amino]acetamide

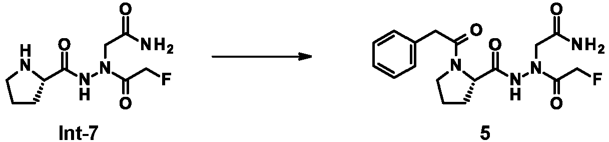

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), phenylacetic acid (28 mg, 0.20 mmol), HBTU (92 mg, 0.24 mmol), and N-methylmorpholine (41 mg, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile, gradient 88:12 to 58:42). After lyophilization, the title compound was isolated as white solid (22 mg, 0.06 mmol, 30% yield). MS (ESI) m/z: 365.1 [M+H]⁺.

### Example 6

### 2-[(2-Fluoroacetyl)-[[(2S)-1-[1-(4-chlorophenyl)cyclopropanecarbonyl]pyrrolidine-2-carbonyl]amino]amino]acetamide

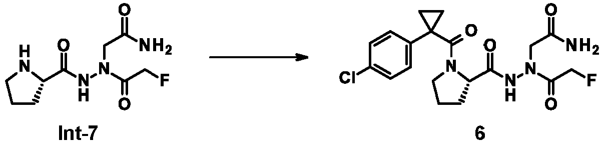

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), 1-(4-chlorophenyl)cyclopropanecarboxylic acid (48 mg, 0.24 mmol), HBTU (92 mg, 0.24 mmol), and N-methylmorpholine (41 mg, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile, gradient 73:27 to 43:57 within 10 min). After lyophilization, the title compound was obtained as white solid (18 mg, 0.04 mmol, 21% yield). MS (ESI) m/z: 425.0 [M+H]⁺.

### Example 7

### 2-[(2-Fluoroacetyl)-[[(2S)-1-(benzenesulfonyl)pyrrolidine-2-carbonyl]amino]amino]acetamide

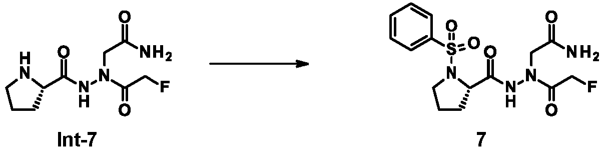

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), benzene sulfone chloride (0.03 mL, 0.22 mmol), and N,N-diisopropylethylamine (0.07 mL, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile). After lyophilization, the title compound was obtained as white solid (29 mg, 0.08 mmol, 37% yield). MS (ESI) m/z: 409.2 [M+Na]⁺.

### Example 8

### 2-[(2-Fluoroacetyl)-[[(2S)-1-(1-phenylcyclopropanecarbonyl)pyrrolidine-2-carbonyl]amino]amino]acetamide

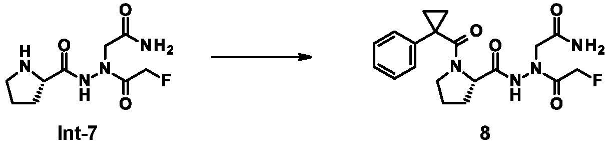

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), 1-phenyl-1-cyclopropanecarboxylic acid (33 mg, 0.20 mmol), HBTU (92 mg, 0.24 mmol), and N-methylmorpholine (41 mg, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile). After lyophilization, the title compound was isolated as white solid (23 mg, 0.06 mmol, 29% yield). MS (ESI) m/z: 413.3 [M+Na]⁺.

### Example 9

### 2-[(2-Fluoroacetyl)-[[(2S)-1-benzylsulfonylpyrrolidine-2-carbonyl]amino]amino]acetamide

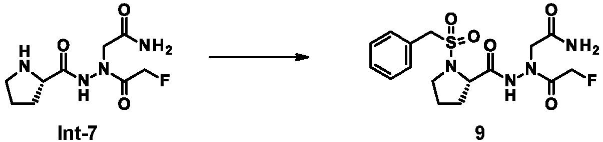

2-[(2-Fluoroacetyl)-[[(2S)-pyrrolidine-2-carbonyl]amino]amino]acetamide (Int-7, 50 mg, 0.20 mmol) was dissolved in dimethylformamide (1.5 mL), alpha-toluenesulfonyl chloride (0.03 mL, 0.22 mmol), and N,N-diisopropylethylamine (0.07 mL, 0.41 mmol) were added. The mixture was stirred at 25°C for 4 h. After that, it was purified directly by preparative HPLC (Phenomenex luna C18 150*25mm* 10um, eluent (water + 0.225% formic acid) / acetonitrile). After lyophilization, the title compound was obtained as white solid (32 mg, 0.08 mmol, 39% yield). MS (ESI) m/z: 423.2 [M+Na]⁺.

### Example 10

### 2-[(2-Fluoroacetyl)-[[(2S)-1-(2-phenylethylsulfonyl)pyrrolidine-2-carbonyl]amino]amino]acetamide

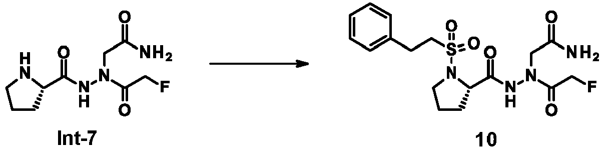

2-[(2-Fluoroacetyl)-[[(2S)-pyrrolidine-2-carbonyl]amino]amino]acetamide (Int-7, 100 mg, 0.41 mmol) was dissolved in dimethylformamide (1.0 mL), 2-phenylethanesulfonyl chloride (83 mg, 0.41 mmol), and N,N-diisopropylethylamine (0.14 mL, 0.81 mmol) were added. The mixture was stirred at 25°C for 4 h. After that, it was purified directly by preparative HPLC (Phenomenex luna C18 150*25mm* 10um, eluent (water + 0.225% formic acid) / acetonitrile). After lyophilization, the title compound was isolated as white solid (40 mg, 0.10 mmol, 24% yield). MS (ESI) m/z: 415.2 [M+H]⁺.

### Example 11

### 2-[(2-Fluoroacetyl)-[[(2S)-1-[1-(4-ethynylphenyl)cyclopropanecarbonyl]pyrrolidine-2-carbonyl]amino]amino]acetamide

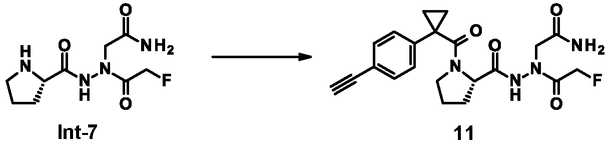

2-[(2-Fluoroacetyl)-[[(2S)-pyrrolidine-2-carbonyl]amino]amino]acetamide (Int-7, 100 mg, 0.41 mmol) was dissolved in dimethylformamide (2 mL), 1-(4-ethynylphenyl)cyclopropanecarboxylic acid (75.6 mg, 0.41 mmol), HBTU (185 mg, 0.49 mmol), and N,N-diisopropylethylamine (0.21 mL, 1.22 mmol) were added. The mixture was stirred at 25°C for 4 h. After that, it was purified directly by preparative HPLC (Phenomenex luna C18 150*25mm* 10um, eluent (water + 0.225% formic acid) / acetonitrile). After lyophilization, the title compound was obtained as white solid (100 mg, 0.24 mmol, 59% yield). MS (ESI) m/z: 415.2 [M+H]⁺.

### Example 12

### 2-[(2-Fluoroacetyl)-[[(2S)-1-[1-[4-(trifluoromethoxy)phenyl]cyclopropanecarbonyl]pyrrolidine-2-carbonyl]amino] amino] acetamide

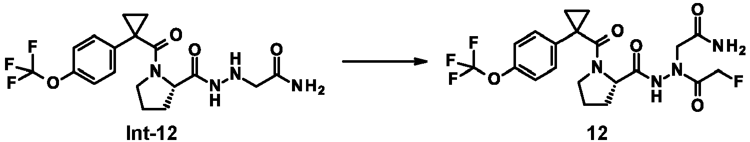

2-(2-((1-(4-(Trifluoromethoxy)phenyl)cyclopropane-1-carbonyl)-L-prolyl)hydrazineyl)acetamide (Int-12, 35 mg, 84 µmol) was dissolved in dichloromethane (1 mL), and pyridine (8.7 mg, 8.8 µl, 110 µmol) was added. The solution was cooled to 0 °C (ice bath), and 2-fluoroacetyl chloride (11 mg, 7.8 µl, 110 µmol) was added. The reaction mixture was stirred at 0 °C for 1 h. After that, additional portions of pyridine (8.7 mg, 8.8 µl, 110 µmol) and 2-fluoroacetyl chloride (11 mg, 7.8 µl, 110 µmol) were added and the stirring was continued at 0 °C for 1 h. Then, it was concentrated in vacuo, the residue was purified by flash chromatography on silica gel (12 g, ethyl acetate / n-heptane, gradient 0:100 to 100:0, then methanol / ethyl acetate, gradient 0:100 to 10:90) to provide the title compound as a white solid (34 mg, 85 % yield). MS (ESI) m/z: 475.2 [M+H]⁺.

### Example 13

### 2-[(2-Fluoroacetyl)-[[(2S)-1-[4-(4-methoxyphenyl)phenyl]sulfonylpyrrolidine-2-carbonyl]amino]amino]acetamide

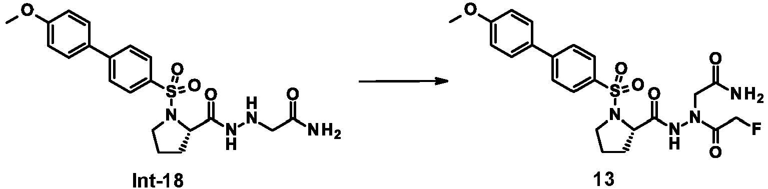

(S)-2-(2-(((4'-Methoxy-[1,1'-biphenyl]-4-yl)sulfonyl)prolyl)hydrazineyl)acetamide (Int-18, 50 mg, 116 µmol) was suspended in dichloromethane (1.5 mL), and pyridine (10.1 mg, 10.2 µl, 127 µmol) was added. The solution was cooled to 0 °C (ice bath), and a solution of 2-fluoroacetyl chloride (12.5 mg, 9.1 µl, 127 µmol) in dichloromethane (100 µl) was added. The reaction mixture was stirred at 0 °C for 30 min. After that, additional portions of pyridine (10.1 mg, 10.2 µl, 127 µmol) and a solution of 2-fluoroacetyl chloride (12.5 mg, 9.1 µl, 127 µmol) in dichloromethane (100 µl) were added and the stirring was continued at 0 °C for 30 min. Then, it was diluted with dichloromethane (5 mL), washed with water (5 mL), the organic layer was dried over sodium sulfate and concentrated in vacuo. The residue was purified by flash chromatography on silica gel (12 g, ethyl acetate / n-heptane, gradient 0:100 to 100:0, then methanol / ethyl acetate, gradient 0:100 to 10:90) to yield, after concentration product containing fractions, the title compound as a white solid (54 mg, 95 % yield). MS (ESI) m/z: 493.2 [M+H]⁺.

### Example 14

### 2-[(2-Fluoroacetyl)-[[(2S)-1-[1-(4-prop-2-ynoxyphenyl)cyclopropanecarbonyl] pyrrolidine-2-carbonyl] amino] amino] acetamide

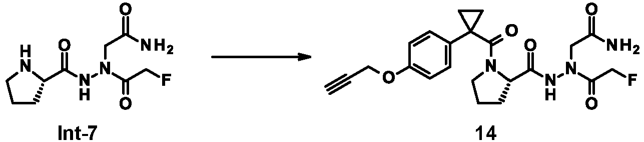

2-[(2-Fluoroacetyl)-[[(2S)-pyrrolidine-2-carbonyl]amino]amino]acetamide (Int-7, 228 mg, 0.92 mmol) was dissolved in dimethylformamide (2 mL), 1-(4-prop-2-ynoxyphenyl)cyclopropanecarboxylic acid (100 mg, 0.46 mmol), HBTU (351 mg, 0.92 mmol), and N,N-diisopropylethylamine (0.24 mL, 1.39 mmol) were added. The mixture was stirred at 20°C for 2 h. After that, it was purified directly by preparative HPLC (Phenomenex luna C18 150*25mm* 10um, eluent (water + 0.225% formic acid) / acetonitrile, gradient 83:17 to 50:50 within 11 min). After lyophilization, the title compound was obtained as white solid (64 mg, 0.13 mmol, 30% yield). MS (ESI) m/z: 445.1 [M+H]⁺.

### Example 15

### 2-[((2R)&(2S)-2-Chloro-2-fluoro-acetyl)-[[(2S)-1-[4-(4-methoxyphenyl)phenyl]sulfonylpyrrolidine-2-carbonyl]amino]amino]acetamide

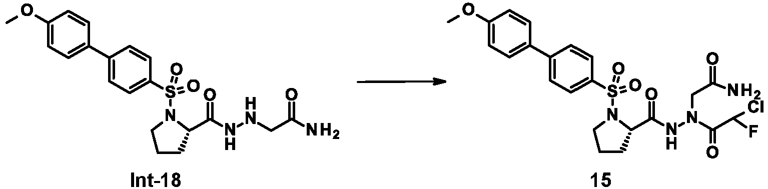

(S)-2-(2-(((4'-Methoxy-[1,1'-biphenyl]-4-yl)sulfonyl)prolyl)hydrazineyl)acetamide (Int-18, 45 mg, 104 µmol) was suspended in dichloromethane (1.5 mL), and pyridine (9.0 mg, 9.2 µl, 114 µmol) was added. The solution was cooled to 0 °C (ice bath), and a solution of 2-chloro-2-fluoroacetyl chloride (15.3 mg, 10.2 µl, 114 µmol) in dichloromethane (100 µl) was added. The reaction mixture was stirred at 0 °C for 30 min. After that, additional portions of pyridine (9.0 mg, 9.2 µl, 114 µmol) and a solution of 2-chloro-2-fluoroacetyl chloride (15.3 mg, 10.2 µl, 114 µmol) in dichloromethane (100 µl) were added and the stirring was continued at 0 °C for 15 min. Then, it was diluted with dichloromethane (5 mL), washed with water (5 mL), the organic layer was dried over sodium sulfate and concentrated in vacuo. The residue was purified by flash chromatography on silica gel (12 g, ethyl acetate / n-heptane, gradient 0:100 to 100:0, then methanol / ethyl acetate, gradient 0:100 to 10:90) to obtain, after concentration product containing fractions, the title compound as a white solid (35 mg, 64 % yield). MS (ESI) m/z: 527.1 [M+H]⁺.

### Example 16

### 2-[((2R)&(2S)-2-Chloro-2-fluoro-acetyl)-[[(2S)-1-[1-[4-(trifluoromethoxy)phenyl]cyclopropanecarbonyl]pyrrolidine-2-carbonyl]amino]amino]acetamide

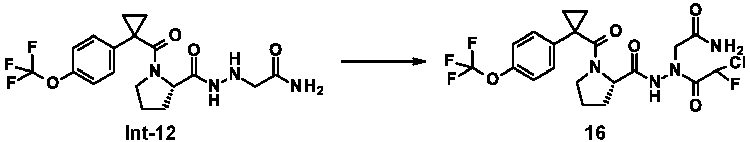

2-(2-((1-(4-(Trifluoromethoxy)phenyl)cyclopropane-1-carbonyl)-L-prolyl)hydrazineyl)acetamide (Int-12, 100 mg, 241 µmol) was dissolved in dichloromethane (2 mL), and pyridine (24.8 mg, 25.2 µl, 314 µmol) was added. The solution was cooled to 0 °C (ice bath), and 2-chloro-2-fluoroacetyl chloride (41.9 mg, 27.9 µl, 314 µmol) was added. The reaction mixture was stirred at 0 °C for 1 h. After that, additional portions of pyridine (24.8 mg, 25.2 µl, 314 µmol) and 2-chloro-2-fluoroacetyl chloride (41.9 mg, 27.9 µl, 314 µmol) were added and the stirring was continued at 0 °C for 1 h. Then, it was concentrated in vacuo, the residue was purified by flash chromatography on silica gel (25 g, ethyl acetate / n-heptane, gradient 0:100 to 100:0, then methanol / ethyl acetate, gradient 0:100 to 10:90) to provide the title compound as a white solid (133 mg, 99 % yield). MS (ESI) m/z: 507.2 [M-H]⁻.

### Example 17

### 2-[(2-Chloroacetyl)-[[(2S)-1-[4-(4-methoxyphenyl)phenyl]sulfonylpyrrolidine-2-carbonyl]amino]amino]acetamide

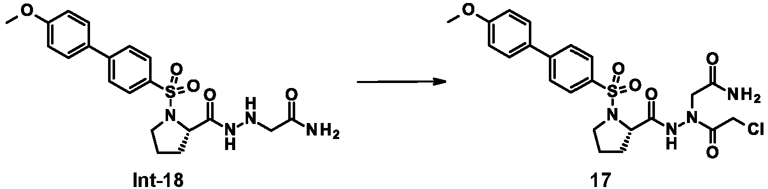

(S)-2-(2-(((4'-Methoxy-[1,1'-biphenyl]-4-yl)sulfonyl)prolyl)hydrazineyl)acetamide (Int-18, 50 mg, 116 µmol) was suspended in dichloromethane (1.5 mL), and pyridine (10.1 mg, 10.2 µl, 127 µmol) was added. The solution was cooled to 0 °C (ice bath), and a solution of 2-chloroacetyl chloride (14.5 mg, 10.2 µl, 127 µmol) in dichloromethane (100 µl) was added. The reaction mixture was stirred at 0 °C for 30 min. Then, it was diluted with dichloromethane (5 mL), washed with water (5 mL), the organic layer was dried over sodium sulfate and concentrated in vacuo. The residue was purified by flash chromatography on silica gel (12 g, ethyl acetate / n-heptane, gradient 0:100 to 100:0, then methanol / ethyl acetate, gradient 0:100 to 20:80) to yield, after concentration product containing fractions, the title compound as a white solid (55 mg, 91 % yield). MS (ESI) m/z: 509.1 [M+H]⁺.

### Example 18

### 2-[(2-Chloroacetyl)-[[(2S)-1-[1-[4-(trifluoromethoxy)phenyl]cyclopropanecarbonyl]pyrrolidine-2-carbonyl]amino]amino]acetamide

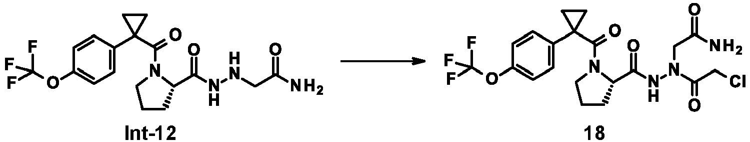

2-(2-((1-(4-(Trifluoromethoxy)phenyl)cyclopropane-1-carbonyl)-L-prolyl)hydrazineyl)acetamide (Int-12, 35 mg, 84 µmol) was dissolved in dichloromethane (1 mL), and pyridine (8.7 mg, 8.8 µl, 110 µmol) was added. The solution was cooled to 0 °C (ice bath), and a solution of 2-chloroacetyl chloride (12.7 mg, 8.9 µl, 110 µmol) in dichloromethane (80 µL) was added. The reaction mixture was stirred at 0 °C for 30 min. After that, additional portions of pyridine (8.7 mg, 8.8 µl, 110 µmol) and a solution of 2-chloroacetyl chloride (12.7 mg, 8.9 µl, 110 µmol) in dichloromethane (80 µL) were added and the stirring was continued at 0 °C for 30 min. Then, it was concentrated in vacuo, the residue was purified by flash chromatography on silica gel (12 g, ethyl acetate / n-heptane, gradient 0:100 to 100:0, then methanol / ethyl acetate, gradient 0:100 to 10:90) to yield the title compound as a white solid (36 mg, 87 % yield). MS (ESI) m/z: 491.2 [M+H]⁺.

### Example 19

### 2-[(2,2-Dichloroacetyl)-[[(2S)-1-[1-[4-(trifluoromethoxy)phenyl]cyclopropanecarbonyl]pyrrolidine-2-carbonyl]amino]amino]acetamide

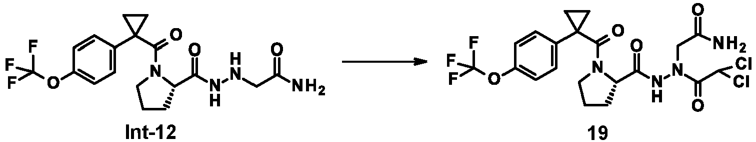

2-(2-((1-(4-(Trifluoromethoxy)phenyl)cyclopropane-1-carbonyl)-L-prolyl)hydrazineyl)acetamide (Int-12, 25 mg, 60 µmol) was dissolved in dichloromethane (1.2 mL), and pyridine (7.2 mg, 7.3 µl, 90 µmol) was added. The solution was cooled to 0 °C (ice bath), and 2,2-dichloroacetyl chloride (13.3 mg, 90 µmol) was added. The reaction mixture was stirred at 0 °C for 3 h. Then, it was concentrated in vacuo, the residue was purified by preparative HPLC (YMC-Triart C18, 12 nm, 5 µm, 100 x 30 mm, acetonitrile / Water + 0.1 % triethylamine, gradient 20:80 to 98:2) to obtain the title compound as a white solid (12 mg, 37 % yield). MS (ESI) m/z: 525.3 [M+H]⁺.

### Example 20

### 2-[(2R)&(2S)-2-Chloropropanoyl-[[(2S)-1-[1-[4-(trifluoromethoxy)phenyl]cyclopropanecarbonyl]pyrrolidine-2-carbonyl]amino]amino]acetamide

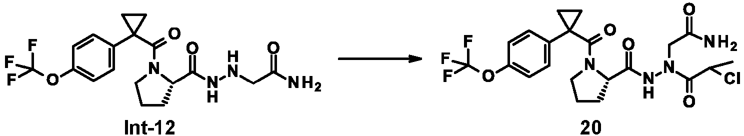

2-(2-((1-(4-(Trifluoromethoxy)phenyl)cyclopropane-1-carbonyl)-L-prolyl)hydrazineyl)acetamide (Int-12, 40 mg, 97 µmol) was dissolved in dichloromethane (1 mL), and pyridine (13.1 mg, 13.3 µl, 166 µmol) was added. The solution was cooled to 0 °C (ice bath), and 2-chloropropionyl chloride (18.4 mg, 14.4 µl, 145 µmol) was added. The reaction mixture was stirred at 0 °C for 1.5 h. Then, it was concentrated in vacuo, the residue was purified by preparative HPLC (YMC-Triart C18, 12 nm, 5 µm, 100 x 30 mm, acetonitrile / Water + 0.1 % triethylamine, gradient 20:80 to 98:2) to obtain the title compound as a white solid (17 mg, 35 % yield). MS (ESI) m/z: 505.3 [M+H]⁺.

### AEP activity enzymatic assay procedure:

The assay uses a fluorogenic substrate, Z-Ala-Ala-Asn-Rh110-(D-Pro) (Biosyntan, Berlin, Ord.Nr. 80773), which fluoresces once it is cleaved by legumain and which can be monitored when excited at 492nm, emission 530nm. Prior to the assay measurements, rhlegumain (in house, construct name: hLGMN(V18-Y433)_VD-8xHis-S2-r) is activated by diluting it from 1.1 mg/ml to 0.1 mg/ml (11-fold) in activation buffer (50 mM sodium acetate, 100 mM NaCl, pH 4.0) and incubation at 37 °C for 2 h. The activated enzyme solution stock is stored at -80°C. For the measurement, the following solutions are dispensed subsequently into 384 well microplates (Corning 384 non-treated, black/clear, Cat. No.: 3540): 8.5 uL assay buffer (20 mM citric acid, 60 mM Na2HPO4, 1 mM EDTA, 0.1% CHAPS, pH 5.8, 0.5 mM TCEP (freshly added)) as negative control or 8.5 uL activated enzyme solution (0.5 nM legumain (final) in assay buffer (20 mM citric acid, 60 mM Na2HPO4, 1 mM EDTA, 0.1% CHAPS, pH 5.8, 0.5 mM TCEP (freshly added))), and 1.5 uL prediluted compound solution (100 uM to 0.1 nM, 1% DMSO in water (final)). After 30 min incubation at 25 °C, 5 uL of substrate solution (0.5 uM Z-Ala-Ala-Asn-Rh110-(D-Pro) (final)) in assay buffer (20 mM citric acid, 60 mM Na2HPO4, 1 mM EDTA, 0.1% CHAPS, pH 5.8, 0.5 mM TCEP (freshly added)) is added and fluorescence is measured on a plate reader, ex 490 nm and em 530 nm, e.g. using PHERAstar or Spectramax instruments in kinetic mode (the first 30 minutes are analyzed).

Results in the AEP activity enzymatic assay are provided for compounds of formula I in Table 1.

**Table 1. Results in AEP activity**

| **Example** | **IC50 (AEP, uM)** | **Example** | **IC50 (AEP, uM)** |
|---|---|---|---|
| 1 | 0.0036 | 11 | 0.0012 |
| 2 | 0.0729 | 12 | 0.0018 |
| 3 | 0.0036 | 13 | 0.0015 |
| 4 | 0.0013 | 14 | 0.0014 |
| 5 | 0.0222 | 15 | 0.0083 |
| 6 | 0.0027 | 16 | 0.0115 |
| 7 | 0.0617 | 17 | 0.0003 |
| 8 | 0.0050 | 18 | 0.0002 |
| 9 | 0.0836 | 19 | 0.0440 |
| 10 | 0.0338 | 20 | 0.0291 |

### Example A

A compound of formula I can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example B

A compound of formula I can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

## Claims

1. Compounds of formula I 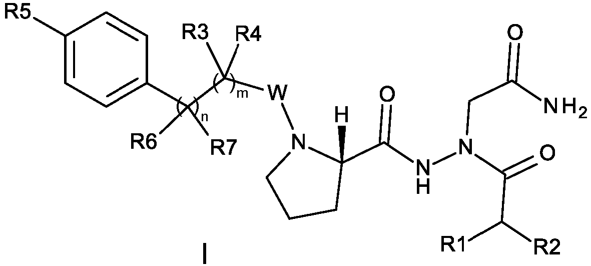 wherein
R¹ is halo or alkyl;
R² is H or halo;
R³ is H or halo;
R⁴ is H or halo;
or R³ and R⁴ form a 3-membered or a 4-membered cycloalkyl ring;
R⁵ is H, halo, haloalkoxy, alkynyl, alkynylalkoxy or alkoxyphenyl;
R⁶ is H;
R⁷ is H;
W is -C(O)- or -S(O)₂-;
m is 0 or 1;
n is 0 or 1;
and pharmaceutically acceptable salts.

2. A compound according to claim 1, wherein R¹ is halo.

3. A compound according to claims 1 or 2, wherein R⁵ is H, halo, or haloalkoxy.

4. A compound according to any of claims 1 to 3, wherein W is -C(O)-.

5. A compound according to any of claims 1 to 4, wherein m is 1 and n is 0.

6. A compound according to any of claims 1 to 5, wherein R³ and R⁴ form a 3-membered or a 4-membered cycloalkyl ring.

7. A compound according to any of claims 1 to 6, selected from
2-[(2-fluoroacetyl)-[[(2S)-1-(2,2-difluoro-2-phenyl-acetyl)pyrrolidine-2-carbonyl]amino]amino]acetamide;
2-[(2-fluoroacetyl)-[[(2S)-1-(3-phenylpropanoyl)pyrrolidine-2-carbonyl]amino]amino]acetamide;
2-[(2-fluoroacetyl)-[[(2S)-1-(1-phenylcyclobutanecarbonyl)pyrrolidine-2-carbonyl]amino]amino]acetamide;
2-[(2-fluoroacetyl)-[[(2S)-1-[1-(4-chlorophenyl)cyclobutanecarbonyl]pyrrolidine-2-carbonyl]amino]amino]acetamide;
2-[(2-fluoroacetyl)-[[(2S)-1-(2-phenylacetyl)pyrrolidine-2-carbonyl]amino]amino]acetamide;
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-(benzenesulfonyl)pyrrolidine-2-carbonyl]amino]amino]acetamide;
2-[(2-fluoroacetyl)-[[(2S)-1-(1-phenylcyclopropanecarbonyl)pyrrolidine-2-carbonyl]amino]amino]acetamide;
2-[(2-fluoroacetyl)-[[(2S)-1-benzylsulfonylpyrrolidine-2-carbonyl]amino]amino]acetamide;
2-[(2-fluoroacetyl)-[[(2S)-1-(2-phenylethylsulfonyl)pyrrolidine-2-carbonyl]amino]amino]acetamide;
2-[(2-fluoroacetyl)-[[(2S)-1-[1-(4-ethynylphenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[(2-fluoroacetyl)-[[(2S)-1-[4-(4-methoxyphenyl)phenyl]sulfonylpyrrolidine-2-carbonyl]amino]amino]acetamide;
2-[(2-fluoroacetyl)-[[(2S)-1-[1-(4-prop-2-ynoxyphenyl)cyclopropanecarbonyl]pyrrolidine-2-carbonyl]amino]amino]acetamide;
2-[((2R)-2-chloro-2-fluoro-acetyl)-[[(2S)-1-[4-(4-methoxyphenyl)phenyl]sulfonylpyrrolidine-2-carbonyl]amino]amino]acetamide;
2-[((2S)-2-chloro-2-fluoro-acetyl)-[[(2S)-1-[4-(4-methoxyphenyl)phenyl]sulfonylpyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[(2-chloroacetyl)-[[(2S)-1-[4-(4-methoxyphenyl)phenyl]sulfonylpyrrolidine-2-carbonyl]amino]amino]acetamide;
2-[(2-chloroacetyl)-[[(2S)-1-[1-[4-(trifluoromethoxy)phenyl]cyclopropanecarbonyl]pyrrolidine-2-carbonyl]amino]amino]acetamide;
2-[(2,2-dichloroacetyl)-[[(2S)-1-[1-[4-(trifluoromethoxy)phenyl]cyclopropanecarbonyl]pyrrolidine-2-carbonyl]amino]amino]acetamide;
2-[(2R)-2-chloropropanoyl-[[(2S)-1-[1-[4-(trifluoromethoxy)phenyl]cyclopropanecarbonyl]pyrrolidine-2-carbonyl]amino]amino]acetamide;
2-[(2S)-2-chloropropanoyl-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof.

8. A compound according to any of claims 1 to 7, selected from
2-[(2-fluoroacetyl)-[[(2S)-1-(2,2-difluoro-2-phenyl-acetyl)pyrrolidine-2-carbonyl]amino]amino]acetamide;
2-[(2-fluoroacetyl)-[[(2S)-1-[1-(4-chlorophenyl)cyclobutanecarbonyl]pyrrolidine-2-carbonyl]amino]amino]acetamide;
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[(2-chloroacetyl)-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof.

9. A compound according to any of claims 1 to 8, selected from
2-[(2-fluoroacetyl)-[[(2S)-1-[1-(4-chlorophenyl)cyclopropanecarbonyl]pyrrolidine-2-carbonyl]amino]amino]acetamide;
2-[(2-fluoroacetyl)-[[(2S)-1-[1-[4-(trifluoromethoxy)phenyl]cyclopropanecarbonyl]pyrrolidine-2-carbonyl]amino]amino]acetamide;
2-[((2R)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
2-[((2S)-2-chloro-2-fluoro-acetyl)-[[(2S)-1-[1-[4-(trifluoromethoxy)phenyl]cyclopropanecarbonyl]pyrrolidine-2-carbonyl]amino]amino]acetamide;
and pharmaceutically acceptable salts thereof.

10. A process to prepare a compound according to any one of claims 1 to 9 comprising the reaction of a compound of formula II with a compound of formula III, wherein R¹, R², R³, R⁴, R⁵, R⁶, R⁷, W, n and m are as defined above and X is an appropriate functional group, selected from F, Cl, Br, OH, O-N-Succinimidyl (OSu).

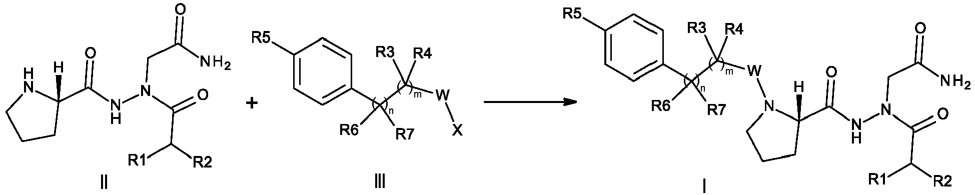

11. A process to prepare a compound according to any one of claims 1 to 9, comprising the reaction of a compound of formula IV with a compound of formula V, wherein R¹, R², R³, R⁴, R⁵, R⁶, R⁷, W, n and m are as defined as above and Y is an appropriate functional group, selected from F, Cl, Br, OH, O-N-Succinimidyl (OSu).

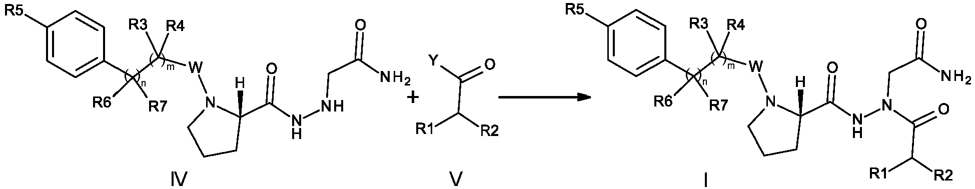

12. A compound according to any one of claims 1 to 9 for use as a therapeutically active substance.

13. A pharmaceutical composition comprising a compound according to any one of claims 1 to 9 and a therapeutically inert carrier.

14. A compound according to any one of claims 1 to 9 for use in the treatment of prophylaxis of Alzheimer's Disease, Primary age-related tauopathy (PART) dementia, Fronto-temporal dementia (FTD-MAPT), Chronic traumatic encephalopathy (CTE), Progressive supranuclear palsy (PSP), Corticobasal degeneration (CBD), Frontotemporal dementia and parkinsonism linked to chromosome 17 (FTDP-17), Lytico-bodig disease (Parkinson-dementia complex of Guam), Ganglioglioma and gangliocytoma, Meningioangiomatosis, Postencephalitic parkinsonism, Subacute sclerosing panencephalitis (SSPE), Pick's disease, or corticobasal degeneration.

15. A compound according to any one of claims 1 to 9 for use in the treatment or prophylaxis of Alzheimer's disease.

## Patentansprüche

1. Verbindungen der Formel I 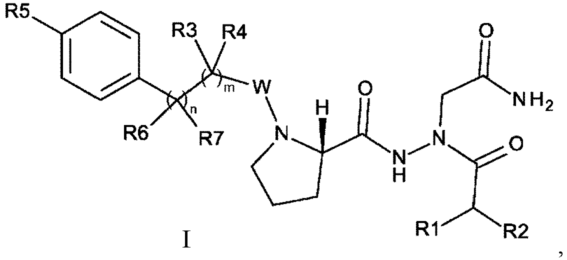 wobei
R¹ Halogen oder Alkyl ist;
R² H oder Halogen ist;
R³ H oder Halogen ist;
R⁴ H oder Halogen ist;
oder R³ und R⁴ einen 3-geliedrigen oder einen 4-geliedrigen Cycloalkylring bilden;
R⁵ H, Halogen, Halogenalkoxy, Alkinyl, Alkinylalkoxy oder Alkoxyphenyl ist;
R⁶ H ist;
R⁷ H ist;
W -C(O)- oder -S(O)₂- ist;
m 0 oder 1 ist;
n 0 oder 1 ist;
und pharmazeutisch unbedenkliche Salze.

2. Verbindung nach Anspruch 1, wobei R¹ Halogen ist.

3. Verbindung nach den Ansprüchen 1 oder 2, wobei R⁵ H, Halogen oder Halogenalkoxy ist.

4. Verbindung nach einem der Ansprüche 1 bis 3, wobei W -C(O)- ist.

5. Verbindung nach einem der Ansprüche 1 bis 4, wobei m 1 ist und n 0 ist.

6. Verbindung nach einem der Ansprüche 1 bis 5, wobei R³ und R⁴ einen 3-geliedrigen oder einen 4-geliedrigen Cycloalkylring bilden.

7. Verbindung nach einem der Ansprüche 1 bis 6, ausgewählt aus
2-[(2-Fluoracetyl)-[[(2S)-1-(2,2-difluor-2-phenylacetyl)pyrrolidin-2-carbonyl]amino]amino]acetamid;
2-[(2-Fluoracetyl)-[[(2S)-1-(3-phenylpropanoyl)pyrrolidin-2-carbonyl]amino]amino]acetamid;
2-[(2-Fluoracetyl)-[[(2S)-1-(1-phenylcyclobutancarbonyl)pyrrolidin-2-carbonyl]amino]amino]acetamid;
2-[(2-Fluoracetyl)-[[(2S)-1-[1-(4-chlorphenyl)cyclobutancarbonyl]pyrrolidin-2-carbonyl]amino]amino]acetamid;
2-[(2-Fluoracetyl)-[[(2S)-1-(2-phenylacetyl)pyrrolidin-2-carbonyl]amino]amino]acetamid;
2-[(2-Fluoracetyl)-[[(2S)-1-[1-(4-chlorphenyl)cyclopropancarbonyl]pyrrolidin-2-carbonyl]amino]amino]acetamid;
2-[(2-Fluoracetyl)-[[(2S)-1-(benzolsulfonyl)pyrrolidin-2-carbonyl]amino]amino]acetamid;
2-[(2-Fluoracetyl)-[[(2S)-1-(1-phenylcyclopropancarbonyl)pyrrolidin-2-carbonyl]amino]amino]acetamid;
2-[(2-Fluoracetyl)-[[(2S)-1-benzylsulfonylpyrrolidin-2-carbonyl]amino]amino]acetamid;
2-[(2-Fluoracetyl)-[[(2S)-1-(2-phenylethylsulfonyl)pyrrolidin-2-carbonyl]amino]amino]acetamid;
2-[(2-Fluoracetyl)-[[(2S)-1-[1-(4-ethinylphenyl)cyclopropancarbonyl]pyrrolidin-2-carbonyl]amino]amino]acetamid;
2-[(2-Fluoracetyl)-[[(2S)-1-[1-[4-(trifluormethoxy)phenyl]cyclopropancarbonyl]pyrrolidin-2-carbonyl]amino]amino]acetamid;
2-[(2-Fluoracetyl)-[[(2S)-1-[4-(4-methoxyphenyl)phenyl]sulfonylpyrrolidin-2-carbonyl]amino]amino]acetamid;
2-[(2-Fluoracetyl)-[[(2S)-1-[1-(4-prop-2-inoxyphenyl)cyclopropancarbonyl]pyrrolidin-2-carbonyl]amino]amino]acetamid;
2-[((2R)-2-Chlor-2-fluoracetyl)-[[(2S)-1-[4-(4-methoxyphenyl)phenyl]sulfonylpyrrolidin-2-carbonyl]amino]amino]acetamid;
2-[((2S)-2-Chlor-2-fluoracetyl)-[[(2S)-1-[4-(4-methoxyphenyl)phenyl]sulfonylpyrrolidin-2-carbonyl]amino]amino]acetamid;
2-[((2R)-2-Chlor-2-fluoracetyl)-[[(2S)-1-[1-[4-(trifluormethoxy)phenyl]cyclopropancarbonyl]pyrrolidin-2-carbonyl]amino]amino]acetamid;
2-[((2S)-2-Chlor-2-fluoracetyl)-[[(2S)-1-[1-[4-(trifluormethoxy)phenyl]cyclopropancarbonyl]pyrrolidin-2-carbonyl]amino]amino]acetamid;
2-[(2-Chloracetyl)-[[(2S)-1-[4-(4-methoxyphenyl)phenyl]sulfonylpyrrolidin-2-carbonyl]amino]amino]acetamid;
2-[(2-Chloracetyl)-[[(2S)-1-[1-[4-(trifluormethoxy)phenyl]cyclopropancarbonyl]pyrrolidin-2-carbonyl]amino]amino]acetamid;
2-[(2,2-Dichloracetyl)-[[(2S)-1-[1-[4-(trifluormethoxy)phenyl]cyclopropancarbonyl]pyrrolidin-2-carbonyl]amino]amino]acetamid;
2-[(2R)-2-Chlorpropanoyl-[[(2S)-1-[1-[4-(trifluormethoxy)phenyl]cyclopropancarbonyl]pyrrolidin-2-carbonyl]amino]amino]acetamid;
2-[(2S)-2-Chlorpropanoyl-[[(2S)-1-[1-[4-(trifluormethoxy)phenyl]cyclopropancarbonyl]pyrrolidin-2-carbonyl]amino]amino]acetamid
und pharmazeutisch unbedenklichen Salzen davon.

8. Verbindung nach einem der Ansprüche 1 bis 7, ausgewählt aus
2-[(2-Fluoracetyl)-[[(2S)-1-(2,2-difluor-2-phenylacetyl)pyrrolidin-2-carbonyl]amino]amino]acetamid;
2-[(2-Fluoracetyl)-[[(2S)-1-[1-(4-chlorphenyl)cyclobutancarbonyl]pyrrolidin-2-carbonyl]amino]amino]acetamid;
2-[(2-Fluoracetyl)-[[(2S)-1-[1-(4-chlorphenyl)cyclopropancarbonyl]pyrrolidin-2-carbonyl]amino]amino]acetamid;
2-[(2-Fluoracetyl)-[[(2S)-1-[1-[4-(trifluormethoxy)phenyl]cyclopropancarbonyl]pyrrolidin-2-carbonyl]amino]amino]acetamid;
2-[((2R)-2-Chlor-2-fluoracetyl)-[[(2S)-1-[1-[4-(trifluormethoxy)phenyl]cyclopropancarbonyl]pyrrolidin-2-carbonyl]amino]amino]acetamid;
2-[((2S)-2-Chlor-2-fluoracetyl)-[[(2S)-1-[1-[4-(trifluormethoxy)phenyl]cyclopropancarbonyl]pyrrolidin-2-carbonyl]amino]amino]acetamid;
2-[(2-Chloracetyl)-[[(2S)-1-[1-[4-(trifluormethoxy)phenyl]cyclopropancarbonyl]pyrrolidin-2-carbonyl]amino]amino]acetamid
und pharmazeutisch unbedenklichen Salzen davon.

9. Verbindung nach einem der Ansprüche 1 bis 8, ausgewählt aus
2-[(2-Fluoracetyl)-[[(2S)-1-[1-(4-chlorphenyl)cyclopropancarbonyl]pyrrolidin-2-carbonyl]amino]amino]acetamid;
2-[(2-Fluoracetyl)-[[(2S)-1-[1-[4-(trifluormethoxy)phenyl]cyclopropancarbonyl]pyrrolidin-2-carbonyl]amino]amino]acetamid;
2-[((2R)-2-Chlor-2-fluoracetyl)-[[(2S)-1-[1-[4-(trifluormethoxy)phenyl]cyclopropancarbonyl]pyrrolidin-2-carbonyl]amino]amino]acetamid;
2-[((2S)-2-Chlor-2-fluoracetyl)-[[(2S)-1-[1-[4-(trifluormethoxy)phenyl]cyclopropancarbonyl]pyrrolidin-2-carbonyl]amino]amino]acetamid
und pharmazeutisch unbedenklichen Salzen davon.

10. Verfahren zur Herstellung einer Verbindung nach einem der Ansprüche 1 bis 9, umfassend die Reaktion einer Verbindung der Formel II mit einer Verbindung der Formel III, wobei R¹, R², R³, R⁴, R⁵, R⁶, R⁷, W, n und m wie oben definiert sind und X eine entsprechende funktionelle Gruppe ist, die aus F, Cl, Br, OH, O-N-Succinimidyl (OSu) ausgewählt ist.

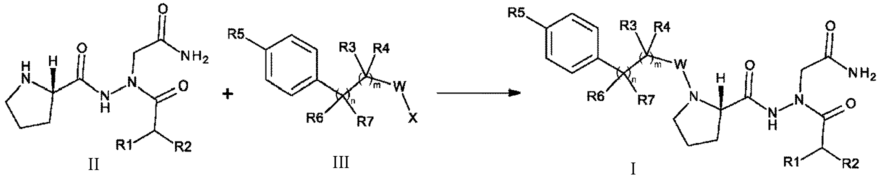

11. Verfahren zur Herstellung einer Verbindung nach einem der Ansprüche 1 bis 9, umfassend die Reaktion einer Verbindung der Formel IV mit einer Verbindung der Formel V, wobei R¹, R², R³, R⁴, R⁵, R⁶, R⁷, W, n und m wie oben definiert sind und Y eine entsprechende funktionelle Gruppe ist, die aus F, Cl, Br, OH, O-N-Succinimidyl (OSu) ausgewählt ist.

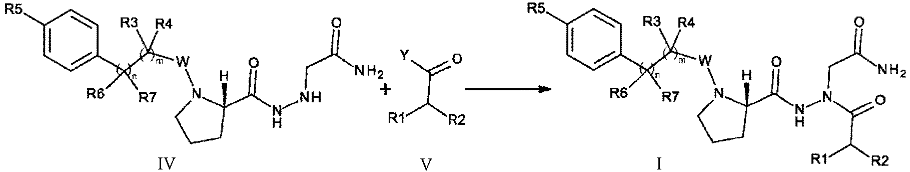

12. Verbindung nach einem der Ansprüche 1 bis 9 zur Verwendung als eine therapeutisch wirksame Substanz.

13. Pharmazeutische Zusammensetzung, umfassend eine Verbindung nach einem der Ansprüche 1 bis 9 und einen therapeutisch inerten Träger.

14. Verbindung nach einem der Ansprüche 1 bis 9 zur Verwendung bei der Behandlung oder Prophylaxe von Alzheimer-Krankheit, primärer altersbedingter Tauopathie (PART)-Demenz, frontotemporaler Demenz (FTD-MAPT), chronisch-traumatischer Enzephalopathie (CTE), progressiver supranukleärer Lähmung (PSP), kortikobasaler Degeneration (CBD), frontotemporaler Demenz und Parkinsonismus, gekoppelt an Chromosom 17 (FTDP-17), Lytico-Bodig-Krankheit (Parkinson-Demenz-Komplex von Guam), Gangliogliom und Gangliozytom, Meningioangiomatose, postenzephalitischem Parkinsonismus, subakuter sklerosierender Panenzephalitis (SSPE), Pick'scher Krankheit oder kortikobasaler Degeneration.

15. Verbindung nach einem der Ansprüche 1 bis 9 zur Verwendung bei der Behandlung oder Prophylaxe von Alzheimer-Krankheit.

## Revendications

1. Composés de formule I 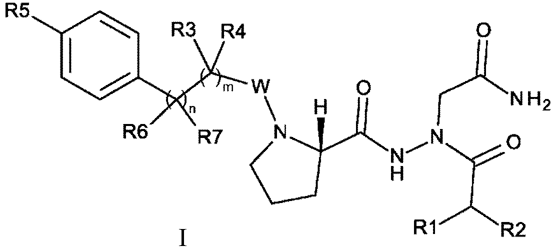 dans lesquels
R¹ représente un halogène ou un alkyle ;
R² représente H ou un halogène ;
R³ représente H ou un halogène ;
R⁴ représente H ou un halogène ;
ou R³ et R⁴ forment un cycle cycloalkyle à 3 chaînons ou 4 chaînons ;
R⁵ représente H, un halogène, un halogénoalcoxy, un alcynyle, un alcynylalcoxy ou un alcoxyphényle ;
R⁶ représente H ;
R⁷ représente H ;
W représente -C(O)- ou -S(O)₂- ;
m représente 0 ou 1 ;
n représente 0 ou 1 ;
et des sels pharmaceutiquement acceptables.

2. Composé selon la revendication 1, dans lequel R¹ représente un halogène.

3. Composé selon les revendications 1 ou 2, dans lequel R⁵ représente H, un halogène ou un halogénoalcoxy.

4. Composé selon l'une quelconque des revendications 1 à 3, dans lequel W représente - C(O)-.

5. Composé selon l'une quelconque des revendications 1 à 4, dans lequel m représente 1 et n représente 0.

6. Composé selon l'une quelconque des revendications 1 à 5, dans lequel R³ et R⁴ forment un cycle cycloalkyle à 3 chainons ou 4 chaînons.

7. Composé selon l'une quelconque des revendications 1 à 6, choisi parmi
le 2-[(2-fluoroacétyl)-[[(2S)-1-(2,2-difluoro-2-phényl-acétyl)pyrrolidine-2-carbonyl]amino]amino]acétamide ;
le 2-[(2-fluoroacétyl)-[[(2S)-1-(3-phénylpropanoyl)pyrrolidine-2-carbonyl]amino]amino]acétamide ;
le 2-[(2-fluoroacétyl)-[[(2S)-1-(1-phénylcyclobutanecarbonyl)pyrrolidine-2-carbonyl]amino]amino]acétamide ;
le 2-[(2-fluoroacétyl)-[[(2S)-1-[1-(4-chlorophényl)cyclobutanecarbonyl]pyrrolidine-2-carbonyl]amino]amino]acétamide ;
le 2-[(2-fluoroacétyl)-[[(2S)-1-(2-phénylacétyl)pyrrolidine-2-carbonyl]amino]amino]acétamide ;
le 2-[(2-fluoroacétyl)-[[(2S)-1-[1-(4-chlorophényl)cyclopropanecarbonyl]pyrrolidine-2-carbonyl]amino]amino]acétamide ;
le 2-[(2-fluoroacétyl)-[[(2S)-1-(benzènesulfonyl)pyrrolidine-2-carbonyl]amino]amino]acétamide ;
le 2-[(2-fluoroacétyl)-[[(2S)-1-(1-phénylcyclopropanecarbonyl)pyrrolidine-2-carbonyl]amino]amino]acétamide ;
le 2-[(2-fluoroacétyl)-[[(2S)-1-benzylsulfonylpyrrolidine-2-carbonyl]amino]amino]acétamide ;
le 2-[(2-fluoroacétyl)-[[(2S)-1-(2-phényléthylsulfonyl)pyrrolidine-2-carbonyl]amino]amino]acétamide ;
le 2-[(2-fluoroacétyl)-[[(2S)-1-[1-(4-éthynylphényl)cyclopropanecarbonyl]pyrrolidine-2-carbonyl]amino]amino]acétamide ;
le 2-[(2-fluoroacétyl)-[[(2S)-1-[1-[4-(trifluorométhoxy)phényl]cyclopropanecarbonyl]pyrrolidine-2-carbonyl]amino]amino]acétamide ;
le 2-[(2-fluoroacétyl)-[[(2S)-1-[4-(4-méthoxyphényl)phényl]sulfonylpyrrolidine-2-carbonyl]amino]amino]acétamide ;
le 2-[(2-fluoroacétyl)-[[(2S)-1-[1-(4-prop-2-ynoxyphényl)cyclopropanecarbonyl]pyrrolidine-2-carbonyl]amino]amino]acétamide ;
le 2-[((2R)-2-chloro-2-fluoro-acétyl)-[[(2S)-1-[4-(4-méthoxyphényl)phényl]sulfonylpyrrolidine-2-carbonyl]amino]amino]acétamide ;
le 2-[((2S)-2-chloro-2-fluoro-acétyl)-[[(2S)-1-[4-(4-méthoxyphényl)phényl]sulfonylpyrrolidine-2-carbonyl]amino]amino]acétamide ;
le 2-[((2R)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
le 2-[((2S)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
le 2-[(2-chloroacétyl)-[[(2S)-1-[4-(4-méthoxyphényl)phényl]sulfonylpyrrolidine-2-carbonyl]amino]amino]acétamide ;
le 2-[(2-chloroacétyl)-[[(2S)-1-[1-[4-(trifluorométhoxy)phényl]cyclopropanecarbonyl]pyrrolidine-2-carbonyl]amino]amino]acétamide ;
le 2-[(2,2-dichloroacétyl)-[[(2S)-1-[1-[4-(trifluorométhoxy)phényl]cyclopropanecarbonyl]pyrrolidine-2-carbonyl]amino]amino]acétamide ;
le 2-[(2R)-2-chloropropanoyl-[[(2S)-1-[1-[4-(trifluorométhoxy)phényl]cyclopropanecarbonyl]pyrrolidine-2-carbonyl]amino]amino]acétamide ;
le 2-[(2S)-2-chloropropanoyl-[[(2S)-1-[1-[4-(trifluorométhoxy)phényl]cyclopropanecarbonyl]pyrrolidine-2-carbonyl]amino]amino]acétamide ;
et des sels pharmaceutiquement acceptables de ceux-ci.

8. Composé selon l'une quelconque des revendications 1 à 7, choisi parmi
le 2-[(2-fluoroacétyl)-[[(2S)-1-(2,2-difluoro-2-phényl-acétyl)pyrrolidine-2-carbonyl]amino]amino]acétamide ;
le 2-[(2-fluoroacétyl)-[[(2S)-1-[1-(4-chlorophényl)cyclobutanecarbonyl]pyrrolidine-2-carbonyl]amino]amino]acétamide ;
le 2-[(2-fluoroacétyl)-[[(2S)-1-[1-(4-chlorophényl)cyclopropanecarbonyl]pyrrolidine-2-carbonyl]amino]amino]acétamide ;
le 2-[(2-fluoroacétyl)-[[(2S)-1-[1-[4-(trifluorométhoxy)phényl]cyclopropanecarbonyl]pyrrolidine-2-carbonyl]amino]amino]acétamide ;
le 2-[((2R)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
le 2-[((2S)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
le 2-[(2-chloroacétyl)-[[(2S)-1-[1-[4-(trifluorométhoxy)phényl]cyclopropanecarbonyl]pyrrolidine-2-carbonyl]amino]amino]acétamide ;
et des sels pharmaceutiquement acceptables de ceux-ci.

9. Composé selon l'une quelconque des revendications 1 à 8, choisi parmi
le 2-[(2-fluoroacétyl)-[[(2S)-1-[1-(4-chlorophényl)cyclopropanecarbonyl]pyrrolidine-2-carbonyl]amino]amino]acétamide ;
le 2-[(2-fluoroacétyl)-[[(2S)-1-[1-[4-(trifluorométhoxy)phényl]cyclopropanecarbonyl]pyrrolidine-2-carbonyl]amino]amino]acétamide ;
le 2-[((2R)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
le 2-[((2S)-2-chloro-2-fluoro-acétyl)-[[(2S)-1-[1-[4-(trifluorométhoxy)phényl]cyclopropanecarbonyl]pyrrolidine-2-carbonyl]amino]amino]acétamide ;
et des sels pharmaceutiquement acceptables de ceux-ci.

10. Procédé de préparation d'un composé selon l'une quelconque des revendications 1 à 9 comprenant la réaction d'un composé de formule II avec un composé de formule III, dans lequel R¹, R², R³, R⁴, R⁵, R⁶, R⁷, W, n et m sont tels que définis ci-dessus et X représente un groupe fonctionnel approprié, choisi parmi F, Cl, Br, OH, O-N-succinimidyle (OSu).

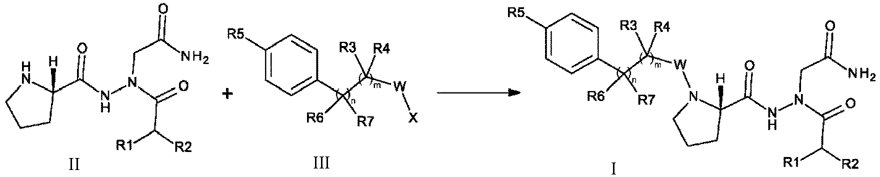

11. Procédé de préparation d'un composé selon l'une quelconque des revendications 1 à 9, comprenant la réaction d'un composé de formule IV avec un composé de formule V, dans lequel R¹, R², R³, R⁴, R⁵, R⁶, R⁷, W, n et m sont tels que définis ci-dessus et Y représente un groupe fonctionnel approprié, choisi parmi F, Cl, Br, OH, O-N-succinimidyle (OSu).

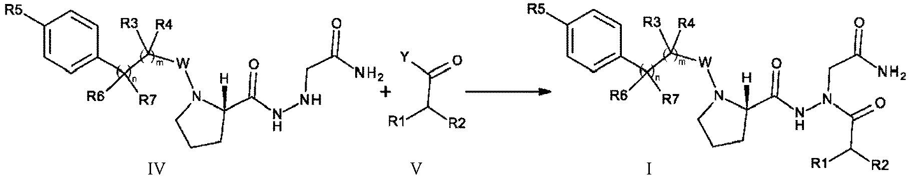

12. Composé selon l'une quelconque des revendications 1 à 9 pour une utilisation comme substance thérapeutiquement active.

13. Composition pharmaceutique comprenant un composé selon l'une quelconque des revendications 1 à 9 et un véhicule thérapeutiquement inerte.

14. Composé selon l'une quelconque des revendications 1 à 9 pour une utilisation dans le traitement ou la prophylaxie de la maladie d'Alzheimer, de la démence de type tauopathie primaire liée à l'âge (PART), de la démence fronto-temporale (FTD-MAPT), de l'encéphalopathie traumatique chronique (CTE), de la paralysie supranucléaire progressive (PSP), de la dégénérescence corticobasale (CBD), de la démence frontotemporale et du parkinsonisme liés au chromosome 17 (FTDP-17), du syndrome de Guam (complexe Parkinson-démence de Guam), du gangliogliome et du gangliocytome, de la méningiomatose, du parkinsonisme post-encéphalique, de la panencéphalite sclérosante subaiguë (SSPE), de la maladie de Pick ou de la dégénérescence corticobasale.

15. Composé selon l'une quelconque des revendications 1 à 9 pour une utilisation dans le traitement ou la prophylaxie de la maladie d'Alzheimer.
